# EUROPEAN PATENT APPLICATION

(11) **EP 4 818 154 A1**
(43) Date of publication of application: **07.10.2026**
(21) Application number: 24897580.7
(22) Date of filing: 27.11.2024
(51) Int. Cl.: C07D 207/26, A61K 31/427, A61K 31/497, A61K 31/4015, A61K 31/4025, A61K 31/4439, A61K 31/4709, A61P 1/00, A61P 1/04, A61P 1/16, A61P 7/00, A61P 9/00, A61P 9/10, A61P 9/12, A61P 11/00, A61P 11/06, A61P 13/12, A61P 17/02, A61P 19/02, A61P 19/04, A61P 25/00

(54) **EP4 AGONIST**

(30) Priority: 28.11.2023 JP 2023200322
(71) Applicant: ONO Pharmaceutical Co., Ltd., Osaka-shi, Osaka 541-8526 (JP)
(72) Inventor: HYAKUTAKE, Ryuichi, Mishima-gun, Osaka 618-8585 (JP); SUZUKI, Takuro, Mishima-gun, Osaka 618-8585 (JP); NAGASHIMA, Nozomu, Mishima-gun, Osaka 618-8585 (JP); IWAKI, Yuzo, Mishima-gun, Osaka 618-8585 (JP)
(74) Representative: Grünecker Patent- und Rechtsanwälte PartG mbB
(86) International application number: PCT/JP2024/041958
(87) International publication number: WO 2025/115896

(57) **Abstract**

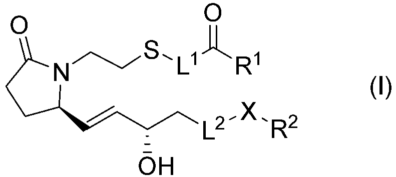

A compound represented by general formula (I) (wherein all symbols have the same meanings as the symbols described in the specification) or a pharmaceutically acceptable salt thereof is useful as an EP4 agonist, particularly has an EP4 agonistic activity, has selectivity for other PGE receptor subtypes, and has a smaller risk of toxicity to the circulatory system. Therefore, the compound or the pharmaceutically acceptable salt thereof is useful as a medicinal ingredient in the prevention and/or treatment of diseases associated with EP4 receptors.

## Description

### TECHNICAL FIELD

The present disclosure relates to a compound having an EP4 agonist action or a pharmaceutically acceptable salt thereof, as well as pharmaceuticals containing them as active ingredients. Specifically, the present disclosure relates to a compound represented by general formula (I): 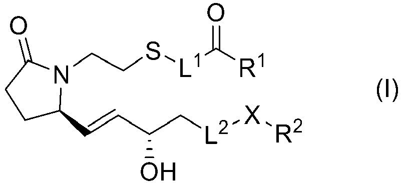 (wherein all symbols represent the same meaning as described later) or a pharmaceutically acceptable salt thereof (hereinafter referred to as a compound of the present disclosure), as well as pharmaceutical compositions containing them as active ingredients.

### BACKGROUND ART

Prostaglandin E2 (abbreviated as PGE2) is known as a metabolite in the arachidonic acid cascade, and its action is known to have a cytoprotective action, uterine contraction, a pain-generating action, promotion of peristaltic movement of the digestive tract, an awakening action, a gastric acid secretion-suppressing action, a blood pressure-lowering action, a diuretic action, and the like.

It is known that there are subtypes having different roles in a PGE receptor, and there are roughly four subtypes known at the present time, which are called EP1, EP2, EP3, and EP4, respectively (Non Patent Literature 1).

Since the EP4 receptor is considered to be involved in suppression of TNF-α production and enhancement of IL-10 production, a compound that binds to the EP4 receptor is considered to be useful for prevention and/or treatment of diseases such as immune diseases (autoimmune diseases such as amyotrophic lateral sclerosis (ALS), multiple sclerosis, Sjögren's syndrome, rheumatoid arthritis, and systemic lupus erythematosus; rejection after organ transplantation; and the like), asthma, neuronal cell death, arthritis, lung injury, pulmonary fibrosis, pulmonary emphysema, bronchitis, chronic obstructive pulmonary disease, liver injury, acute hepatitis, nephritis (acute nephritis, chronic nephritis), renal failure, hypertension, myocardial ischemia, systemic inflammatory response syndrome, sepsis, hemophagocytic syndrome, macrophage activation syndrome, Still's disease, Kawasaki disease, burn injury, systemic granuloma, inflammatory bowel disease (ulcerative colitis, Crohn's disease, and the like), hypercytokinemia during dialysis, multiple organ failure, and shock. The EP4 receptor is also related to a mucosa-protecting action (Non Patent Literature 2), and is considered to be useful for prevention and/or treatment of gastrointestinal ulcers such as gastric ulcers and duodenal ulcers and stomatitis.

The compound that selectively binds to EP4 has no pain generation that is considered to be caused by EP1, no uterine contraction action that is considered to be caused by EP3, and the like, and thus is considered to be a drug that has no influence on these pain generation and uterine contraction action.

Many compounds having an EP4 agonist action have been found so far, and when systemic administration such as oral administration or intravenous administration is performed, there is a concern about influences on the circulatory system such as a decrease in blood pressure and an increase in heart rate (Non Patent Literature 3).

On the other hand, Patent Literature 1 describes that the compound represented by the following general formula (A) is a compound having EP4 agonist activity.

General formula (A) is 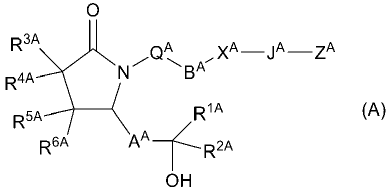
wherein Q^{A} is CH₂ or an oxygen atom;
B^{A} is a -CH₂- group, a -(CH₂)₂- group, a -(CH₂)₃- group, a -(CH₂)₄- group, a -(CH₂)₅-group, a -CH=CH- group, a -CH₂-CH=CH- group, a CH=CH-CH₂- group, or a -CH₂-CH=CH-CH₂- group, provided that when B^{A} is a -CH=CH- group or a -CH=CH-CH₂- group, Q^{A} is -CH₂- group;
X^{A} is an -NR^{aA}- group (wherein R^{aA} is a hydrogen atom, a halogen atom, an alkyl group, or an acyl group), an -O- group, an -S- group, an -SO- group, an -SO₂- group, or a single bond, provided that when X^{A} is a single bond, Q^{A} is an oxygen atom;
J^{A} is a -(CR^{bA}R^{cA})_{nA}- group (wherein nA is an integer of 1 to 4, and R^{bA} and R^{cA} are both hydrogen atoms, or one or both of R^{bA} and R^{cA} are lower alkyl groups, and the rest are hydrogen atoms, or R^{bA} and R^{cA}, when bonded to the same atom, form a C2-C5-polymethylene group), or a -CH₂-CH=CH- group;
A^{A} is a -CH₂-CH₂- group, a -CH=CH- group, or a -C=C- group;
Z^{A} is a CH₂OH group, a -C(O)OR' group, and a -C(O)NR'R" group, a -C(O)NSO₂R' group, a -P(Cl-C6)alkyl(O)(OR') group, a(-PO(OR')₂ group, or a tetrazol-5-yl group, wherein R' and R" are each independently a hydrogen atom or an alkyl group;
nA is 1, 2, 3, or 4;
R^{1A} is a -(CH₂)_{pA}R^{7A} group or a -(CH₂)_{qA}OR^{8A} group, wherein R^{7A} and R^{8A} are each independently an alkyl group, a haloalkyl group, a cycloalkyl group, a heterocyclyl group, an aryl group, or a heteroaryl group;
pA and qA are each independently 0, 1, 2, 3, 4, or 5;
R^{2A} is a hydrogen atom, an alkyl group, an alkenyl group, or an alkynyl group; and
R^{3A}, R^{4A}, R^{5A}, and R^{6A} each independently represent a hydrogen atom or an alkyl group.

The compound represented by the following general formula (B) in Patent Literature 2 is described as a compound having EP4 agonist activity.

General formula (B) is 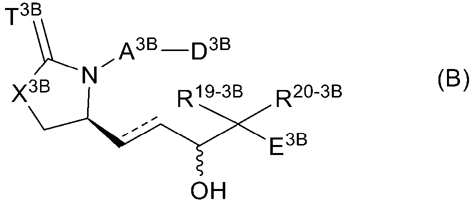
wherein R^{19-3B} and R^{20-3B} each independently represent a hydrogen atom, an alkyl group, or a halogen atom;
T^{3B} represents an oxygen atom or a sulfur atom;
X^{3B} represents a -CH₂- group, an -O- group, or an -S- group;
A^{3B} represents A^{B} or A^{2-3B};
A^{1-3B} represents an alkylene group, an alkenylene group, or an alkynylene group that may be substituted with an alkyl group, and A^{2-3B} represents a -G^{1-3B}-G^{2-3B}-G^{3-3B}- group;
G^{1-3B} represents an alkylene group or the like, G^{2-3B} represents a -Y^{3B}- group, a -(ring 1^{3B})- group, a -Y^{3B}-(ring 1^{3B})- group or the like, and Y^{3B} represents an -S- group or the like;
G^{3-3B} represents a single bond, an alkylene group, an alkenylene group, an alkynylene group, or the like;
D^{3B} represents D^{1-3B} or D^{2-3B};
D^{1-3B} represents a -COOH group, a -COOR^{2-3B} group, a tetrazol-5-yl group, or a CONR^{3-3B}SO₂R^{4-3B} group, and D^{2-3B} represents a -CH₂OH group, a -CH₂OR^{5-3B} group, a hydroxyl group, an -OR^{5-3B} group, a formyl group, a -CONR^{6-3B}R^{7-3B} group, a -CONR^{6-3B}SO₂R^{8-3B} group, or the like;
E^{3B} represents E^{1-3B} or E^{2-3B};
E^{1-3B} represents a cycloalkyl group or ring 3^{3B}, and E^{2-3B} represents a cycloalkyl group, ring 4^{3B}, or ring 5^{3B};
ring 1^{3B} and ring 5^{3B} may be substituted with R^{21-3B} and/or R^{22-3B};
ring 3^{3B} may be substituted with R^{21-3B};
ring 4^{3B} is necessarily substituted with one R^{22-3B}, and may be further substituted with R^{21-3B} and/or R^{22-3B};
R^{21-3B} represents an alkyl group, an alkoxy group, a halogen atom, a phenyl group, or the like;
R^{22-3B} represents an alkenyl group, an alkynyl group, an alkylthio group, a hydroxyl group, an -NR^{24-3B}R^{25-3B} group, an alkyl group substituted with an alkoxy group, an alkyl group substituted with an alkoxy group substituted with a halogen atom, an alkyl group substituted with an -NR^{24-3B}R^{25-3B} group, ring 6^{3B}, -O-ring 7^{3B}, an alkyl group substituted with ring 7^{3B}, an alkoxy group substituted with ring 7^{3B}, an alkyl group substituted with -O-ring 7^{3B}, a -COOR^{26-3B} group, or the like;
ring 1^{3B}, ring 5^{3B}, ring 6^{3B}, and ring 7^{3B} represent
a C3-15 monocyclic, bicyclic, or tricyclic carbocyclic aryl that may be partially or fully saturated, or
a 3- to 15-membered monocyclic, bicyclic, or tricyclic heterocyclic aryl that may be partially or fully saturated containing 1 to 4 heteroatoms selected from an oxygen atom, a nitrogen atom, and a sulfur atom; and
ring 3^{3B} and ring 4^{3B} represent a thienyl group, a phenyl group, or a furyl group (The definition of groups was partially excerpted).

The compound represented by the following general formula (C) in Patent Literature 3 is described as a compound having EP2 and EP4 agonist activity.

General formula (C) is 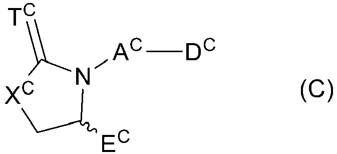
wherein T^{C} represents an oxygen atom or a sulfur atom;
X^{C} represents a -CH₂- group, an -O- group, or an -S- group;
A^{C} represents A^{1C} or A^{2C};
A^{1C} represents an alkylene group, an alkenylene group, or an alkynylene group that may be substituted with an alkyl group, and A^{2C} represents a -G^{1C}-G^{2C}-G^{3C}- group;
G^{1C} represents an alkylene group or the like, G^{2C} represents a -Y^{C}- group, a -ring 1^{C}-group, a -Y^{C}-ring 1^{C}- group or the like, Y^{C} represents an -S- group or the like, and G^{3C} represents a single bond, an alkylene group, an alkenylene group, an alkynylene group, or the like;
D^{C} represents D^{1C} or D^{2C};
D^{1C} represents a -COOH group, a -COOR^{2C} group, a tetrazol-5-yl group, or a - CONR^{3C}SO₂R^{4C} group, and D^{2C} represents a -CH₂OH group, a -CH₂OR^{5C} group, a hydroxyl group, an -OR^{5C} group, a formyl group, a -CONR^{6C}R^{7C} group, a -CONR^{6C}SO₂R^{8C} group, or the like;
E^{C} represents E^{1C} or E^{2C};
E^{1C} represents 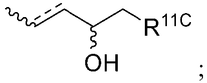
R^{11C} represents an alkyl group, an alkylthio group, an alkyl group substituted with a cycloalkyl group, an alkyl group substituted with ring 2^{C}, or an alkyl group substituted with - W^{1C}-W^{2C}-ring 2^{C};
W^{1C} represents an -O- group, an -S- group, an -SO- group, an -SO₂- group, an -NR^{11-1C}- group, a carbonyl group, an -NR^{11-1C}SO₂- group, a carbonylamino group, or an aminocarbonyl group, and W^{2C} represents a single bond or an alkyl group that may be substituted;
ring 1^{C} or ring 2^{C} may be substituted with 1 to 5 R^{C}s;
R^{C} represents an alkyl group, an alkenyl group, an alkynyl group, an alkoxy group, an alkylthio group, a halogen atom, a hydroxyl group, a nitro group, an -NR^{15C}R^{16C} group, an alkyl group substituted with an alkoxy group, an alkyl group substituted with a halogen atom, an alkyl group substituted with an alkoxy group substituted with a halogen atom, an alkyl group substituted with an -NR^{15C}R^{16C} group, a ring 5^{C} group, an -O-ring 5^{C} group, an alkyl group substituted with a ring 5^{C} group, an alkenyl group substituted with a ring 5^{C} group, an alkynyl group substituted with a ring 5^{C} group, an alkoxy group substituted with a ring 5^{C} group, an alkyl group substituted with an -O-ring 5^{C} group, a COOR^{17C} group, an alkoxy group substituted with a halogen atom, a formyl group, an alkyl group substituted with a hydroxy group, or an acyl group;
ring 5^{C} may be substituted with a substituent selected from the following;
an alkyl group, an alkenyl group, an alkynyl group, an alkoxy group, an alkyl group substituted with an alkoxy group, a halogen atom, a hydroxyl group, an alkyl group substituted with a halogen atom, and an alkyl group substituted with an alkoxy group substituted with a halogen atom; and
ring 1^{C}, ring 2^{C}, and ring 5^{C} each independently represent
(1) C3-15 monocyclic, bicyclic, or tricyclic carbocyclic aryl that may be partially or fully saturated, or (2) 3- to 15-membered monocyclic, bicyclic, or tricyclic heterocyclic aryl that may be partially or fully saturated containing a heteroatom selected from 1 to 4 nitrogen atoms, 1 to 2 oxygen atoms, and/or 1 to 2 sulfur atoms (The definition of groups was partially excerpted).

The compound represented by the following general formula (D) in Patent Literature 4 is described as a compound having EP2 and EP4 agonist activity.

General formula (D) is 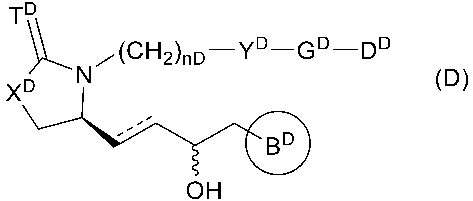
wherein D^{D} represents -COOR^{1D} or a tetrazolyl group, R^{1D} represents a hydrogen atom or an alkyl group, G^{D} represents ring A^{D} or an alkylene group, and ring A^{D} represents 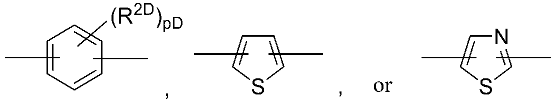
(wherein R^{2D} represents a halogen atom, an alkyl group, or an alkoxy group), Y^{D} represents a bond or -S-, T^{D} represents an oxygen atom or a sulfur atom, X^{D} represents -CH₂-, -O-, or -S-, and ring B^{D} represents a C3-7 cycloalkyl group that may have a substituent 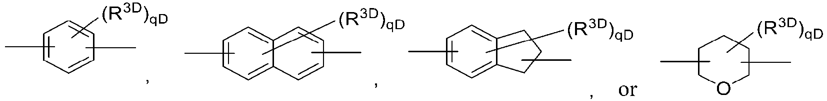
(wherein R^{3D} represents a halogen atom, an alkyl group that may be substituted with a halogen atom, an alkoxy group that may be substituted with a halogen atom, an alkyl group substituted with an alkoxy group, a phenyl group, or a 3- to 15-membered monocyclic, bicyclic, or tricyclic heterocyclic ring that may be partially or fully saturated, and the phenyl group or the heterocyclic ring in R^{3D} may be substituted with a halogen atom, an alkyl group, an alkoxy group, and/or a nitro group), and nD represents an integer of 1 to 4 (The definition of groups was partially excerpted).

The compound represented by the following general formula (E) in Patent Literature 5 is described as a compound having EP4 agonist activity.

General formula (E) is 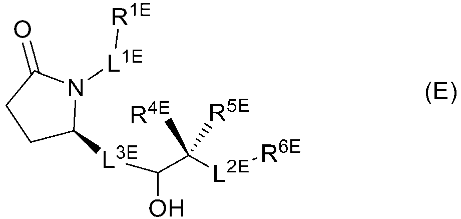
wherein L^{1E} represents
   a)an alkylene group, an alkenylene group, or an alkynylene group, wherein the alkylene group, the alkenylene group, or the alkynylene group may be optionally substituted with 1 to 4 fluorine substituents;
   b)-(CH₂)_{tE}-G^{E}-(CH₂)_{pE}-, wherein tE represents 0 to 2, pE represents 0 to 3, and tE + pE = 0 to 4; or
   c)-(CH₂)_{nE}-G^{1E}-(CH₂)_{pE}-, -(CH₂)_{nE}-G^{2E}-(CH₂)_{pE}-, -(CH₂)_{nE}-C≡C-G^{2E}-, or -(CH₂)_{nE}-C(R^{12E})=C(R^{12E})-G^{2E}-, wherein nE represents 1 to 5, pE represents 0 to 3, and nE + pE = 1 to 6;
G^{E} represents 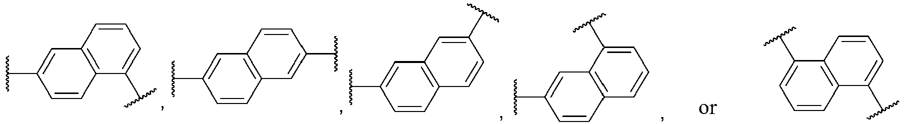
G^{1E} represents O, CO, S, SO, SO₂, or NR^{7E};
G^{2E} represents 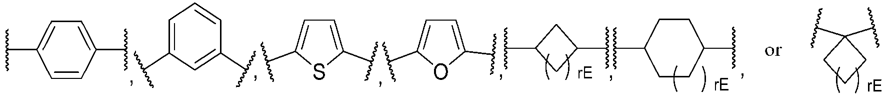
wherein G^{2E} may be optionally substituted with 1 to 3 substituents selected from the group consisting of an alkyl group, a haloalkyl group, a cyano group, a halogen atom, an alkoxy group, and a haloalkoxy group;
R^{1E} represents a COOR^{9E} group, a CONR^{9E}R^{10E} group, a CH₂OR^{9E} group, a SO₃R^{9E} group, a SO₂NR^{9E}R^{10E} group, a PO(OR^{9E})₂ group, or a tetrazol-5-yl group;
L^{3E} represents a -C(R^{2E})₂-C(R^{3E})₂- group, -C(R^{2E})=C(R^{3E})- group, -C=C- group, or 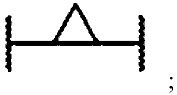
R^{4E} and R^{5E} each independently represent H or an alkyl group, or R^{4E} and R^{5E} may be taken together with the carbon atom to which they are bonded to form a cycloalkyl group;
L^{2E} represents an alkylene group or an alkenylene group, wherein the alkylene group and the alkenylene group may be optionally substituted with 1 to 4 fluorine substituents;
R^{6E} represents an aryl group or a heteroaryl group, wherein the aryl group or the heteroaryl group may be optionally substituted with 1 to 4 substituents selected from the group consisting of an alkyl group, a haloalkyl group, a cyano group, a halogen atom, an alkoxy group, a haloalkoxy group, and an -alkylene-alkoxy group; and
rE represents 0 or 1 (The definition of groups was partially excerpted).

### CITATIONS LIST

### PATENT LITERATURE

Patent Literature 1: WO 2003/007941 A
Patent Literature 2: WO 2003/009872 A
Patent Literature 3: WO 2003/074483 A
Patent Literature 4: WO 2004/065365 A
Patent Literature 5: WO 2014/144610 A

### NON PATENT LITERATURE

Non Patent Literature 1: J. Lipid Mediators Cell Signaling, 379-391 (1995)
Non Patent Literature 2: The Journal of Clinical Investigation, 883-893 (2002)
Non Patent Literature 3: International Heart Journal, 107-114 (2017)

### SUMMARY OF INVENTION

### TECHNICAL PROBLEMS

An object of the present disclosure includes providing a compound useful as an EP4 agonist, in particular, a compound having EP4 agonist activity, selectivity for other PGE receptor subtypes, and a low risk of toxicity to the circulatory system.

### SOLUTIONS TO PROBLEMS

As a result of intensive studies to solve the above problems, the present inventors have found that a compound represented by general formula (I) described later is useful as an EP4 agonist, in particular, it has a characteristic of having a low risk of toxicity to the circulatory system because of excellent large intestine selectivity despite having potent EP4 agonist activity.

The present disclosure, in one aspect, provides embodiments of:
[1] a compound represented by general formula (I): 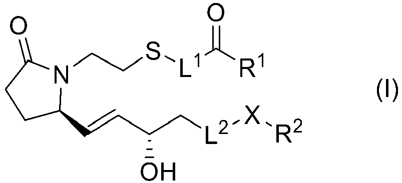 wherein
   R¹ represents (1) an -OH group, (2) an -NHCH₂CH₂SO₃H group, (3) an - NHCH₂COOH group, or (4) an -NHCH(COOH)CH₂CH₂COOH group;
   L¹ represents (1) a thiazole ring or (2) C1-4 alkylene;
   L² represents (1) C3-5 alkylene or (2) a benzene ring;
   X represents (1) -CO-, (2) -NR^{X}-, or (3) a sulfur atom;
   R^{X} represents (1) a hydrogen atom or (2) a C1-4 alkyl group;
   R² represents (1) a methyl group, (2) a benzene ring, (3) a 5- to 10-membered heterocyclic ring, (4) a -COR²⁻¹ group, (5) a -CO-(benzene ring) group, (6) a -CO-(5- to 10-membered heterocyclic ring) group, (7) an -NH-(benzene ring) group, or (8) an -OH group;
   when R² represents a benzene ring, a 5- to 10-membered heterocyclic ring, a -CO-(benzene ring) group, a -CO-(5- to 10-membered heterocyclic ring) group, or an -NH-(benzene ring) group, the benzene ring or the 5- to 10-membered heterocyclic ring may be substituted with 1 to 5 R²⁻²s;
   R²⁻¹ represents (1) a C1-4 alkyl group that may be substituted with a C1-4 alkoxy group or (2) an -NH-(C1-4 alkyl) group; and
   R²⁻² represents (1) a halogen atom, (2) a C1-4 alkyl group, (3) a C1-4 haloalkyl group, (4) a C1-4 alkoxy group, (5) a C1-4 haloalkoxy group, or (6) a benzene ring; or a pharmaceutically acceptable salt thereof;
[2] A pharmaceutical composition comprising the compound according to [1] or a pharmaceutically acceptable salt thereof as an active ingredient, and further comprising a pharmaceutically acceptable carrier;
[3] the pharmaceutical composition according to [2], which is an EP4 agonist; and the like.

### ADVANTAGEOUS EFFECTS OF INVENTION

The compound of the present disclosure is useful as an EP4 agonist, in particular, it has EP4 agonist activity and selectivity for other PGE receptor subtypes. Furthermore, the compound of the present disclosure is excellent in large intestine selectivity and thus has a low risk of toxicity to the circulatory system, and therefore the compound of the present disclosure is useful as a preventive and/or therapeutic agent for a disease related to an EP4 receptor.

### DESCRIPTION OF EMBODIMENTS

Hereinafter, the present disclosure will be described in detail.

In the present specification, examples of a halogen atom include fluorine, chlorine, bromine, and iodine atoms.

In the present specification, examples of a C1-4 alkyl group include methyl, ethyl, propyl, isopropyl, butyl, isobutyl, sec-butyl, and tert-butyl groups.

In the present specification, examples of a C3-5 alkyl group include propyl, isopropyl, butyl, isobutyl, sec-butyl, tert-butyl, pentyl, isopentyl, sec-pentyl, tert-pentyl, and neopentyl groups, and isomers thereof.

In the present specification, examples of a C1-4 alkylene include methylene, ethylene, propylene (trimethylene), butylene (tetramethylene), isopropylene, isobutylene, and isomers thereof.

In the present specification, examples of a C3-5 alkylene include propylene (trimethylene), butylene (tetramethylene), pentylene (pentamethylene), isopropylene, isobutylene, isopentylene, and isomers thereof.

In the present specification, a C1-4 haloalkyl group means, for example, an alkyl group substituted with one or more halogen atoms, and specific examples thereof include a fluoromethyl group, a chloromethyl group, a bromomethyl group, an iodomethyl group, a difluoromethyl group, a trifluoromethyl group, a 1-fluoroethyl group, a 2-fluoroethyl group, a 2-chloroethyl group, a pentafluoroethyl group, a 1-fluoropropyl group, a 2-chloropropyl group, a 3-fluoropropyl group, a 3-chloropropyl group, a 4,4,4-trifluorobutyl group, a 4-bromobutyl group, and isomers thereof.

In the present specification, examples of a C1-4 alkoxy group include methoxy, ethoxy, propoxy, isopropoxy, butoxy, isobutoxy, sec-butoxy, and tert-butoxy groups.

In the present specification, a C1-4 haloalkoxy group means, for example, an alkoxy group substituted with one or more halogen atoms, and specific examples thereof include a fluoromethoxy group, a chloromethoxy group, a bromomethoxy group, an iodomethoxy group, a difluoromethoxy group, a trifluoromethoxy group, a 1-fluoroethoxy group, a 2-fluoroethoxy group, a 2-chloroethoxy group, a pentafluoroethoxy group, a 1-fluoropropoxy group, a 2-chloropropoxy group, a 3-fluoropropoxy group, a 3-chloropropoxy group, a 4,4,4-trifluorobutoxy group, a 4-bromobutoxy group, and isomers thereof.

In the present specification, a 5- to 6-membered heterocyclic ring may be a nonaromatic heterocyclic ring or an aromatic heterocyclic ring. The 5- to 6-membered heterocyclic ring may have, for example, at least one heteroatom selected from an oxygen atom, a sulfur atom, and a nitrogen atom as a ring constituting atom. Examples of a 5- to 6-membered heterocyclic ring include pyrrole, imidazole, triazole, tetrazole, pyrazole, pyridine, pyrazine, pyrimidine, pyridazine, furan, pyran, thiophene, thiopyran, oxazole, isoxazole, thiazole, isothiazole, furazan, oxadiazole, oxazine, oxadiazine, thiadiazole, thiazine, thiadiazine, pyrroline, pyrrolidine, imidazoline, imidazolidine, triazoline, triazolidine, tetrazoline, tetrazolidine, pyrazoline, pyrazolidine, dihydropyridine, tetrahydropyridine, piperidine, dihydropyrazine, tetrahydropyrazine, piperazine, dihydropyrimidine, tetrahydropyrimidine, perhydropyrimidine, dihydropyridazine, tetrahydropyridazine, perhydropyridazine, dihydrofuran, tetrahydrofuran, dihydropyran, tetrahydropyran, dihydrothiophene, tetrahydrothiophene, dihydrothiopyran, tetrahydrothiopyran, dihydrooxazole, tetrahydrooxazole (oxazolidine), dihydroisoxazole, tetrahydroisoxazole (isoxazolidine), dihydrothiazole, tetrahydrothiazole (thiazolidine), dihydroisothiazole, tetrahydroisothiazole (isothiazolidine), dihydrofurazan, tetrahydrofurazan, dihydrooxadiazole, tetrahydrooxadiazole (oxadiazolidine), dihydrooxazine, tetrahydrooxazine, dihydrooxadiazine, tetrahydrooxadiazine, dihydrothiadiazole, tetrahydrothiadiazole (thiadiazolidine), dihydrothiazine, tetrahydrothiazine, dihydrothiadiazine, tetrahydrothiadiazine, morpholine, thiomorpholine, oxathiane, dioxolane, dioxane, dithiolane, and dithiane rings.

In the present specification, a 5- to 10-membered heterocyclic ring may be a nonaromatic heterocyclic ring or an aromatic heterocyclic ring. The 5- to 10-membered heterocyclic ring may be a monocyclic heterocyclic ring or a fused cyclic heterocyclic ring such as a 2-4 cyclic heterocyclic ring. The 5- to 10-membered heterocycle may have, for example, at least one heteroatom selected from an oxygen atom, a sulfur atom, and a nitrogen atom as a ring constituting atom. Examples of a 5- to 10-membered heterocyclic ring include pyrrole, imidazole, triazole, tetrazole, pyrazole, pyridine, pyrazine, pyrimidine, pyridazine, furan, pyran, thiophene, thiopyran, oxazole, isoxazole, thiazole, isothiazole, furazan, oxadiazole, oxazine, oxadiazine, thiadiazole, thiazine, thiadiazine, pyrroline, pyrrolidine, imidazoline, imidazolidine, triazoline, triazolidine, tetrazoline, tetrazolidine, pyrazoline, pyrazolidine, dihydropyridine, tetrahydropyridine, piperidine, dihydropyrazine, tetrahydropyrazine, piperazine, dihydropyrimidine, tetrahydropyrimidine, perhydropyrimidine, dihydropyridazine, tetrahydropyridazine, perhydropyridazine, dihydrofuran, tetrahydrofuran, dihydropyran, tetrahydropyran, dihydrothiophene, tetrahydrothiophene, dihydrothiopyran, tetrahydrothiopyran, dihydrooxazole, tetrahydrooxazole (oxazolidine), dihydroisoxazole, tetrahydroisoxazole (isoxazolidine), dihydrothiazole, tetrahydrothiazole (thiazolidine), dihydroisothiazole, tetrahydroisothiazole (isothiazolidine), dihydrofurazan, tetrahydrofurazan, dihydrooxadiazole, tetrahydrooxadiazole (oxadiazolidine), dihydrooxazine, tetrahydrooxazine, dihydrooxadiazine, tetrahydrooxadiazine, dihydrothiadiazole, tetrahydrothiadiazole (thiadiazolidine), dihydrothiazine, tetrahydrothiazine, dihydrothiadiazine, tetrahydrothiadiazine, morpholine, thiomorpholine, oxathiane, dioxolane, dioxane, dithiolane, dithiane, azepine, diazepine, oxepine, thiepin, oxazepine, oxadiazepine, thiazepine, thiadiazepine, indole, isoindole, indolizine, benzofuran, isobenzofuran, benzothiophene, isobenzothiophene, dithianaphthalene, indazole, quinoline, isoquinoline, quinolizine, purine, phthalazine, pteridine, naphthyridine, quinoxaline, quinazoline, cinnoline, benzoxazole, benzothiazole, benzimidazole, chromene, benzofurazan, benzothiadiazole, benzotriazole, dihydroazepine, tetrahydroazepine, perhydroazepine, dihydrodiazepine, tetrahydrodiazepine, perhydrodiazepine, dihydrooxepine, tetrahydrooxepine, perhydrooxepine, dihydrothiepin, tetrahydrothiepin, perhydrothiepin, dihydrooxazepine, tetrahydrooxazepine, perhydrooxazepine, dihydrooxadiazepine, tetrahydrooxadiazepine, perhydrooxadiazepine, dihydrothiazepine, tetrahydrothiazepine, perhydrothiazepine, dihydrothiadiazepine, tetrahydrothiadiazepine, perhydrothiadiazepine, indoline, isoindoline, dihydrobenzofuran, perhydrobenzofuran, dihydroisobenzofuran, perhydroisobenzofuran, dihydrobenzothiophene, perhydrobenzothiophene, dihydroisobenzothiophene, perhydroisobenzothiophene, dihydroindazole, perhydroindazole, dihydroquinoline, tetrahydroquinoline, perhydroquinoline, dihydroisoquinoline, tetrahydroisoquinoline, perhydroisoquinoline, dihydrophthalazine, tetrahydrophthalazine, perhydrophthalazine, dihydronaphthyridine, tetrahydronaphthyridine, perhydronaphthyridine, dihydroquinoxaline, tetrahydroquinoxaline, perhydroxyquinoxaline, dihydroquinazoline, tetrahydroquinazoline, perhydroquinazoline, dihydrocinnoline, tetrahydrocinnoline, perhydrocinnoline, benzoxathiane, dihydrobenzoxazine, dihydrobenzothiazine, pyrazinomorpholine, dihydrobenzoxazole, perhydrobenzoxazole, dihydrobenzothiazole, perhydrobenzothiazole, dihydrobenzimidazole, perhydrobenzimidazole, dioxaindane, benzodioxane, chromane, benzodithiolane, benzodithiane, azaspiro[4.4]nonane, oxazaspiro[4.4]nonane, dioxaspiro[4.4]nonane, azaspiro[4.5]decane, thiaspiro[4.5]decane, dithiaspiro[4.5]decane, dioxaspiro[4.5]decane, oxazaspiro[4.5]decane, azabicyclo[3.2.1]octane, oxabicyclo[3.2.1]octane, thieno[3,2-c]pyrazole, thieno[2,3-c]pyrazole, thieno[2,3-d]thiazole, thieno[2,3-d][1.2.3]triazole, dihydropyrano[3,4-d]thiazole, dihydrothieno[2,3-b]pyran, dihydrothieno[3,2-c]pyran, dihydrothieno[3,2-b]pyran, dihydrothieno[3,2-c]thiopyran, tetrahydrothieno[3,2-b]pyridine, tetrahydrothieno[3,2-c]pyridine, and thieno[3,2-c]pyridine rings.

In the present disclosure, R¹ represents an -OH group, an -NHCH₂CH₂SO₃H group, an -NHCH₂COOH group, or an -NHCH(COOH)CH₂CH₂COOH group. R¹ is preferably an - OH group an -NHCH₂CH₂SO₃H group or an -NHCH(COOH)CH₂CH₂COOH group, and more preferably an -OH group or an -NHCH₂CH₂SO₃H group.

In the present disclosure, L¹ represents a thiazole ring, or a C1-4 alkylene. L¹ is preferably a thiazole ring, and a substitution pattern of the thiazole ring is preferably 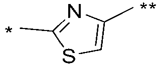 wherein * represents a bonding site with a sulfur atom, and ** represents a bonding site with -COR¹.

In the present disclosure, L² represents a C3-5 alkylene, or a benzene ring. L² is preferably propylene (trimethylene), butylene (tetramethylene), pentylene (pentamethylene), or a benzene ring, and more preferably butylene (tetramethylene) or a benzene ring.

In the present disclosure, X represents a -CO-, an -NR^{X}-, or a sulfur atom. X is preferably an -NR^{X}- or a sulfur atom, and more preferably a sulfur atom.

In the present disclosure, R^{X} represents a hydrogen atom, or a C1-4 alkyl group. R^{X} is preferably a hydrogen atom or a methyl group.

In the present disclosure, R² represents a methyl group, a benzene ring, a 5- to 10-membered heterocyclic ring, a -COR²⁻¹ group, a -CO-(benzene ring) group, a -CO-(5- to 10-membered heterocyclic ring) group, an -NH-(benzene ring) group, or an -OH group. R² is preferably a benzene ring, a 5- to 10-membered heterocyclic ring, a -COR²⁻¹ group, a -CO-(benzene ring) group, a -CO-(5- to 10-membered heterocyclic ring) group, or an -NH-(benzene ring) group, more preferably a benzene ring, a 5- to 10-membered heterocyclic ring, a -CO-(benzene ring) group, or a -CO-(5- to 10-membered heterocyclic ring) group, still more preferably a benzene ring, a 5- to 6-membered heterocyclic ring, a -CO-(benzene ring) group, or a -CO-(5- to 6-membered heterocyclic ring) group, even more preferably a benzene ring, a pyridine ring, a pyrazine ring, a pyrazole ring, an imidazole ring, a thiazole ring, a triazole ring, a tetrazole ring, a quinoline ring, a -CO-(benzene ring) group, a -CO-(pyridine ring) group, a -CO-(pyrazine ring) group, or a -CO-(furan ring) group, and particularly preferred is a benzene ring, a pyridine ring, a pyrazine ring, or a -CO-(benzene ring) group.

R² may also be selected depending on the type of X. For example, when X is -CO-, R² is preferably a methyl group, a benzene ring, a 5- to 10-membered heterocyclic ring, an - NH- (benzene ring) group, or an -OH group, and more preferably an -NH-(benzene ring) group. When X is -NR^{X}-, R² is preferably a methyl group, a benzene ring, a 5- to 10-membered heterocyclic ring, a -COR²⁻¹ group, a -CO-(benzene ring) group, or a -CO-(5- to 10-membered heterocyclic ring) group, more preferably a benzene ring, a -COR²⁻¹ group, a - CO-(benzene ring) group, or a -CO-(5- to 10-membered heterocyclic ring) group, and still more preferably a benzene ring, a -COCH₂OCH₃ group, a -CONHCH₂CH₃ group, a -CO-(benzene ring) group, a -CO-(pyridine ring) group, a -CO-(pyrazine ring) group, or a -CO-(furan ring) group. When X is a sulfur atom, R² is preferably a methyl group, a benzene ring or a 5- to 10-membered heterocyclic ring, more preferably a benzene ring or a 5- to 10-membered heterocyclic ring, still more preferably a 5- to 10-membered heterocyclic ring, and even more preferably a pyridine ring, a pyrazine ring, a pyrazole ring, a thiazole ring, an imidazole ring, a triazole ring, a tetrazole ring or a quinoline ring.

In the present disclosure, the 5- to 10-membered heterocyclic ring is preferably a pyridine ring, a pyrazine ring, a pyrazole ring, a pyrimidine ring, an imidazole ring, a thiazole ring, a triazole ring, a tetrazole ring, a furan ring, or a quinoline ring, more preferably a pyridine ring or a pyrazine ring, a pyrimidine ring, an imidazole ring, a thiazole ring, a triazole ring, a tetrazole ring, a furan ring, or a quinoline ring, and still more preferably a pyridine ring or a pyrazine ring.

In the present disclosure, R²⁻¹ represents a C1-4 alkyl group which may be substituted by a C1-4 alkoxy group or an -NH-(C1-4 alkyl) group. R²⁻¹ is preferably a C1-4 alkyl group substituted with a C1-4 alkoxy group or an -NH-(C1-4 alkyl) group, and more preferably a -CH₂OCH₃ group or an -NHCH₂CH₃ group.

In the present disclosure, R²⁻² represents a halogen atom, a C1-4 alkyl group, a C1-4 haloalkyl group, a C1-4 alkoxy group, a C1-4 haloalkoxy group, or a benzene ring. R²⁻² is preferably a halogen atom, a C1-4 alkyl group, a C1-4 alkoxy group, a C1-4 haloalkoxy group, or a benzene ring, more preferably a fluorine atom, a chlorine atom, a methyl group, a methoxy group, a difluoromethoxy group, or a benzene ring, and still more preferably a fluorine atom, a methyl group, or a methoxy group.

In the present disclosure, a compound represented by general formula (I) is preferably a compound represented by general formula (I-1): 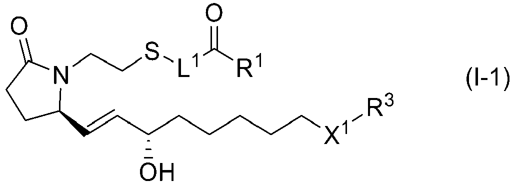
wherein R³ represents (1) a benzene ring, (2) a pyridine ring, (3) a pyrazine ring, (4) a pyrimidine ring, (5) an imidazole ring, (6) a thiazole ring, (7) a triazole ring, (8) a tetrazole ring, (9) a furan ring, (10) a quinoline ring, or (11) a pyrazole ring;
R³ may be substituted with 1 to 5 R³⁻¹s;
R³⁻¹ represents (1) a halogen atom, (2) a C1-4 alkyl group, (3) a C1-4 haloalkyl group, (4) a C1-4 alkoxy group, (5) a C1-4 haloalkoxy group, or (6) a benzene ring;
X¹ represents (1) -NR^{X}- or (2) a sulfur atom; and
the other symbols represent the same meaning as described above; more preferably a compound represented by general formula (I-2): 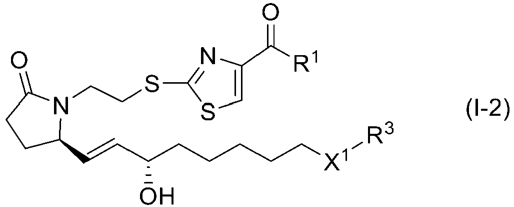
wherein all symbols represent the same meaning as described above; and still more preferably a compound represented by general formula (I-3): 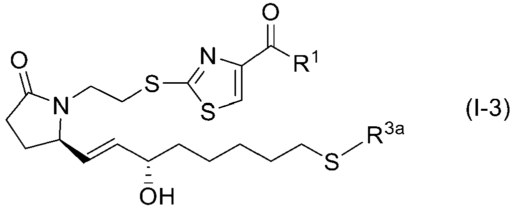
wherein R^{3a} represents (1) a pyridine ring or (2) a pyrazine ring, R^{3a} may be substituted with 1 to 3 R³⁻¹s, and the other symbols represent the same meaning as described above.

In the present disclosure, R³ is preferably a benzene ring, a pyridine ring, a pyrazine ring, a pyrazole ring, a thiazole ring, an imidazole ring, a triazole ring, a tetrazole ring, or a quinoline ring, more preferably a benzene ring, a pyridine ring, or a pyrazine ring, and still more preferably a pyridine ring or a pyrazine ring.

In the present disclosure, R³⁻¹ is preferably a halogen atom, a C1-4 alkyl group, a C1-4 alkoxy group, a C1-4 haloalkoxy group, or a benzene ring, more preferably a fluorine atom, a chlorine atom, a methyl group, a methoxy group, a difluoromethoxy group, or a benzene ring, and still more preferably a fluorine atom, a methyl group, or a methoxy group.

In the present disclosure, another aspect of the compound represented by general formula (I) is preferably a compound represented by general formula (I-4): 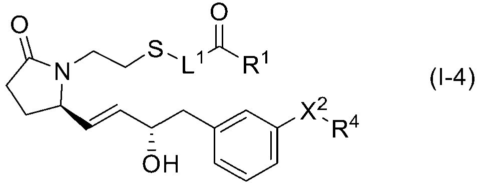
wherein R⁴ represents (1) a -COR⁴⁻¹ group, (2) a -CO-(benzene ring) group, (3) a - CO-(pyridine ring) group, (4) a -CO-(pyrazine ring) group, (5) a -CO-(furan ring) group, (6) an -NH-(benzene ring) group, or (7) an -OH group;
when R⁴ represents a -CO-(benzene ring) group, a -CO-(pyridine ring) group, a -CO-(pyrazine ring) group, a -CO-(furan ring) group, or an -NH-(benzene ring) group, the benzene ring, the pyridine ring, the pyrazine ring, or the furan ring may be substituted with 1 to 5 R⁴⁻²s;
R⁴⁻¹ represents (1) a C1-4 alkyl group that may be substituted with a C1-4 alkoxy group or (2) an -NH-(C1-4 alkyl) group;
R⁴⁻² represents (1) a halogen atom, (2) a C1-4 alkyl group, (3) a C1-4 haloalkyl group, (4) a C1-4 alkoxy group, (5) a C1-4 haloalkoxy group, or (6) a benzene ring;
X² represents (1) -CO- or (2) -NR^{X}-; and
the other symbols represent the same meaning as described above, or a compound represented by general formula (I-5):

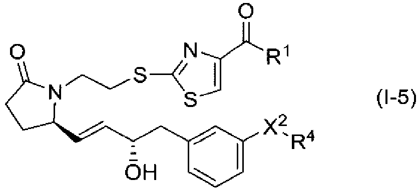

Wherein all symbols represent the same meaning as described above.

In the present disclosure, R⁴ is preferably a -CO-(benzene ring) group, a -CO-(pyridine ring) group, or a -CO-(pyrazine ring) group, more preferably a -CO-(benzene ring) group, or a -CO-(pyridine ring) group, and still more preferably a -CO-(benzene ring) group.

In the present disclosure, R⁴⁻² is preferably a C1-4 alkyl group or a C1-4 alkoxy group, and more preferably a methyl group or a methoxy group.

In the present disclosure, the compound represented by general formula (I) is preferably a combination of preferred definitions of R¹, R², L¹, L², and X described above.

In the present disclosure, another aspect of the compound represented by general formula (I) is most preferably an example compound described in Examples described later or a pharmaceutically acceptable salt thereof.

In the present disclosure, all isomers are included unless otherwise indicated. For example, an alkyl group, an alkoxy group, an alkylene group, and the like include a linear group and a branched group. Furthermore, isomers (E, Z, cis, and trans forms) in a double bond, a ring, and a fused ring; isomers (R and S forms, α and β forms, enantiomer, diastereomer) due to the presence of an asymmetric carbon; optically active substances (D, L, d, and l forms) having optical rotation; polar substances (high-polar substance, low-polar substance) obtained by chromatographic separation; equilibrium compounds; rotational isomers; mixtures of these in any proportion; and racemic mixtures are all included in the present disclosure. In addition, the present disclosure also includes all isomers by tautomerism.

In the present disclosure, unless otherwise specified, as apparent to those skilled in the art, symbol 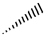 represents bonding to the other side of the paper surface (that is, α-configuration), symbol 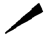 represents bonding to the front side of the paper surface (that is, β-configuration), and symbol 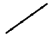 represents any mixture of α- and β-configurations.

### [Salt]

The compound represented by general formula (I) is converted into a salt by a known method.

The salt is a pharmaceutically acceptable salt.

The salt is preferably water-soluble.

Examples of the pharmaceutically acceptable salt include acid addition salts, alkali metal salts, alkaline earth metal salts, ammonium salts, and amine salts.

Examples of the acid addition salt include inorganic acid salts such as hydrochloride, hydrobromide, hydroiodide, sulfate, phosphate, and nitrate; and organic acid salts such as acetate, lactate, tartrate, benzoate, citrate, methanesulfonate, ethanesulfonate, trifluoroacetate, benzenesulfonate, toluenesulfonate, isethionate, glucuronate, and gluconate.

Examples of the alkali metal salt include potassium salts and sodium salts.

Examples of the alkaline earth metal salt include calcium salts and magnesium salts.

Examples of the ammonium salt include tetramethylammonium salts.

Examples of the amine salt include triethylamine salts, methylamine salts, dimethylamine salts, cyclopentylamine salts, benzylamine salts, phenethylamine salts, piperidine salts, monoethanolamine salts, diethanolamine salts, tris(hydroxymethyl)aminomethane salts, lysine salts, arginine salts, and N-methyl-D-glucamine salts.

The compound represented by general formula (I) can be converted into an N-oxide by any method. The N-oxide represents a compound obtained by oxidizing a nitrogen atom of the compound represented by general formula (I).

The compound represented by general formula (I) and a pharmaceutically acceptable salt thereof may exist in a non-solvated form, or may exist in a solvated form with a pharmaceutically acceptable solvent such as water or ethanol. The solvate is preferably a hydrate. The compound represented by general formula (I) and a pharmaceutically acceptable salt thereof can be converted into a solvate.

The compound represented by general formula (I) can form a cocrystal with an appropriate cocrystal former. The cocrystal is preferably a pharmaceutically acceptable one formed with a pharmaceutically acceptable cocrystal former. The cocrystal is typically defined as a crystal in which two or more different molecules are formed by intermolecular interactions that are different from ionic bonds. The cocrystal may be a complex of a neutral molecule and a salt. The cocrystal can be prepared by known methods, for example, by melting crystallization, by recrystallization from a solvent, or by physically grinding the components together. Suitable cocrystal formers include those described in WO 2006/007448 A.

In the present disclosure, all references to the compound of the present disclosure include the compound represented by general formula (I), a pharmaceutically acceptable salt thereof, an N-oxide thereof, a solvate thereof (for example, a hydrate), or a cocrystal thereof; or an N-oxide of a pharmaceutically acceptable salt of the compound represented by general formula (I), a solvate thereof (for example, a hydrate), or a cocrystal thereof.

### [Prodrug]

A prodrug of the compound represented by general formula (I) refers to a compound that is converted into the compound represented by general formula (I) by a reaction with an enzyme, gastric acid, or the like in vivo. Examples of the prodrug of the compound represented by general formula (I) include a compound in which, when the compound represented by general formula (I) has an amino group, the amino group is acylated, alkylated, or phosphorylated (for example, a compound in which the amino group of the compound represented by general formula (I) is eicosanoylated, alanylated, pentylaminocarbonylated, (5-methyl-2-oxo-1,3-dioxolen-4-yl)methoxycarbonylated, tetrahydrofuranylated, pyrrolidylmethylated, pivaloyloxymethylated, acetoxymethylated, tertbutylated, or the like); a compound in which, when the compound represented by general formula (I) has a hydroxyl group, the hydroxyl group is acylated, alkylated, phosphorylated, or borated (for example, a compound in which the hydroxyl group of the compound represented by general formula (I) is acetylated, palmitoylated, propanoylated, pivaloylated, succinylated, fumarylated, alanylated, dimethylaminomethylcarbonylated, or the like); and a compound in which, when the compound represented by general formula (I) has a carboxy group, the carboxy group is esterified or amidated (for example, a compound in which the carboxy group of the compound represented by general formula (I) is ethylesterified, phenylesterified, carboxymethylesterified, dimethylaminomethylesterified, pivaloyloxymethylesterified, 1- {(ethoxycarbonyl)oxy}ethylesterified, phthalidylesterified, (5-methyl-2-oxo-1,3-dioxolen-4-yl)methylesterified, 1-{[(cyclohexyloxy)carbonyl]oxy}ethylesterified, or methylamidated, a compound amidated with an amino acid (for example, taurine, glutamic acid, or the like), or the like. These compounds can be produced by a method known per se. The prodrug of the compound represented by general formula (I) may be either a hydrate or a non-hydrate. In addition, the prodrug of the compound represented by general formula (I) may be one that changes to the compound represented by general formula (I) under physiological conditions as described in "Pharmaceutical Research and Development", published in 1990 by Hirokawa Shoten Co., Vol. 7, "Molecular Design", pp 163-198.

Furthermore, each atom constituting the compound represented by general formula (I) may be substituted with an isotope thereof (for example, ²H, ³H, ¹¹C, ¹³C, ¹⁴C, ¹⁵N, ¹⁶N, ¹⁷O, ¹⁸O, ¹⁸F, ³⁵S, ³⁶Cl, ⁷⁷Br, ¹²⁵I, and the like) or the like.

In the present specification, large intestine selectivity means a ratio of a large intestine concentration to a plasma concentration at the time of administration of a compound (large intestine concentration/plasma concentration).

### [Method for producing compound of the present disclosure]

The compound of the present disclosure can be produced by appropriately improving a known method, for example, a method shown below, a method equivalent thereto, a method described in Comprehensive Organic Transformations: A Guide to Functional Group Preparations, 3rd Edition (Richard C. Larock, John Wiley & Sons Inc, 2018), a method shown in Examples, or the like, and using a combination thereof. As a starting material, a salt may be used. The order of performing each reaction can be appropriately changed depending on the introduced protecting group and reaction conditions.

In addition, a compound having an amino group, a carboxyl group, or a hydroxyl group can be produced by performing a known deprotection reaction after an appropriate reaction step, if necessary, using a compound protected with a protecting group commonly used for these groups, for example, a protecting group described in T. W. Greene, Protective Groups in Organic Synthesis, Wiley, New York, 5th Edition, 2014.

Examples of the protecting group of the carboxyl group include methyl, ethyl, tert-butyl, trichloroethyl, benzyl (Bn), phenacyl, p-methoxybenzyl, trityl, and 2-chlorotrityl.

Examples of the protecting group of the amino group or the tetrazolyl group include a benzyloxycarbonyl group, a tert-butoxycarbonyl group, an allyloxycarbonyl (Alloc) group, a 1-methyl-1-(4-biphenyl)ethoxycarbonyl (Bpoc) group, a trifluoroacetyl group, a 9-fluorenylmethoxycarbonyl group, a benzyl (Bn) group, a p-methoxybenzyl group, a benzyloxymethyl (BOM) group, and a 2-(trimethylsilyl)ethoxymethyl (SEM) group.

Examples of the protecting group of the hydroxyl group or hydroxamic acid include methyl, trityl, methoxymethyl (MOM), 1-ethoxyethyl (EE), methoxyethoxymethyl (MEM), 2-tetrahydropyranyl (THP), trimethylsilyl (TMS), triethylsilyl (TES), tert-butyldimethylsilyl (TBDMS), tert-butyldiphenylsilyl (TBDPS), acetyl (Ac), pivaloyl, benzoyl, benzyl (Bn), p-methoxybenzyl, allyloxycarbonyl (Alloc), and 2,2,2-trichloroethoxycarbonyl (Troc).

The deprotection reaction is known, and can be performed by the following method. For example,
(1) a deprotection reaction by alkaline hydrolysis,
(2) a deprotection reaction under acidic conditions,
(3) a deprotection reaction by hydrogenolysis,
(4) a deprotection reaction of a silyl group,
(5) a deprotection reaction using a metal,
(6) a deprotection reaction using a metal complex, and the like are exemplified.

These methods are specifically described as follows:
(1) The deprotection reaction by alkaline hydrolysis is performed, for example, in an organic solvent (for example, methanol, tetrahydrofuran (THF), dioxane, or the like), using a hydroxide of an alkali metal (for example, sodium hydroxide, potassium hydroxide, lithium hydroxide, or the like), a hydroxide of an alkaline earth metal (for example, barium hydroxide, calcium hydroxide, or the like), a carbonate (for example, sodium carbonate, potassium carbonate, or the like), an aqueous solution thereof, or a mixture thereof at 0 to 40°C.
(2) The deprotection reaction under acidic conditions is performed, for example, in an organic solvent (for example, dichloromethane, chloroform, dioxane, ethyl acetate, methanol, isopropyl alcohol, THF, anisole, or the like), in an organic acid (for example, acetic acid, trifluoroacetic acid, methanesulfonic acid, p-tosylic acid, or the like), an inorganic acid (for example, hydrochloric acid, sulfuric acid, or the like), or a mixture thereof (for example, hydrogen bromide/acetic acid or the like), in the presence or absence of 2,2,2-trifluoroethanol at 0 to 100°C.
(3) The deprotection reaction by hydrogenolysis is performed, for example, in a solvent (for example, an ether-based solvent (for example, THF, dioxane, dimethoxyethane, diethyl ether, or the like), an alcohol-based solvent (for example, methanol, ethanol, or the like), a benzene-based solvent (for example, benzene, toluene, or the like), a ketone-based solvent (for example, acetone, methyl ethyl ketone, or the like), a nitrile-based solvent (for example, acetonitrile or the like), an amide-based solvent (for example, N,N-dimethylformamide (DMF) or the like), water, ethyl acetate, acetic acid, or a mixed solvent of two or more thereof, or the like), in the presence of a catalyst (for example, palladium-carbon, palladium black, palladium hydroxide-carbon, platinum oxide, Raney nickel, or the like), in a hydrogen atmosphere under normal pressure or pressure, or in the presence of ammonium formate at 0 to 200°C.
(4) The deprotection reaction of a silyl group is performed, for example, in an organic solvent (for example, THF, acetonitrile, or the like) miscible with water, using tetrabutylammonium fluoride at 0 to 40°C. It is also performed, for example, in an organic acid (for example, acetic acid, trifluoroacetic acid, methanesulfonic acid, p-tosylic acid, or the like), an inorganic acid (for example, hydrochloric acid, sulfuric acid, or the like), or a mixture thereof (for example, hydrogen bromide/acetic acid or the like) at -10 to 100°C.
(5) The deprotection reaction using a metal is performed, for example, in an acidic solvent (for example, acetic acid, a buffer having a pH of 4.2 to 7.2, or a mixed solution of these solutions and an organic solvent such as THF), in the presence of powdered zinc while applying ultrasonic waves if necessary at 0 to 40°C.
(6) The deprotection reaction using a metal complex is performed, for example, in an organic solvent (for example, dichloromethane, DMF, THF, ethyl acetate, acetonitrile, dioxane, ethanol, or the like), water, or a mixed solvent thereof, in the presence of a trapping reagent (for example, tributyltin hydride, triethylsilane, dimedone, morpholine, diethylamine, pyrrolidine, or the like), an organic acid (for example, acetic acid, formic acid, 2-ethylhexanoic acid, or the like), and/or an organic acid salt (for example, sodium 2-ethylhexanoate, potassium 2-ethylhexanoate, or the like), in the presence or absence of a phosphine reagent (for example, triphenylphosphine or the like), using a metal complex (for example, tetrakistriphenylphosphine palladium(0), bis(triphenylphosphine)palladium(II) dichloride, palladium(II) acetate, tris(triphenylphosphine)rhodium(I) chloride, or the like) at 0 to 40°C.

The compound represented by general formula (I) can be produced by Reaction Scheme 1.

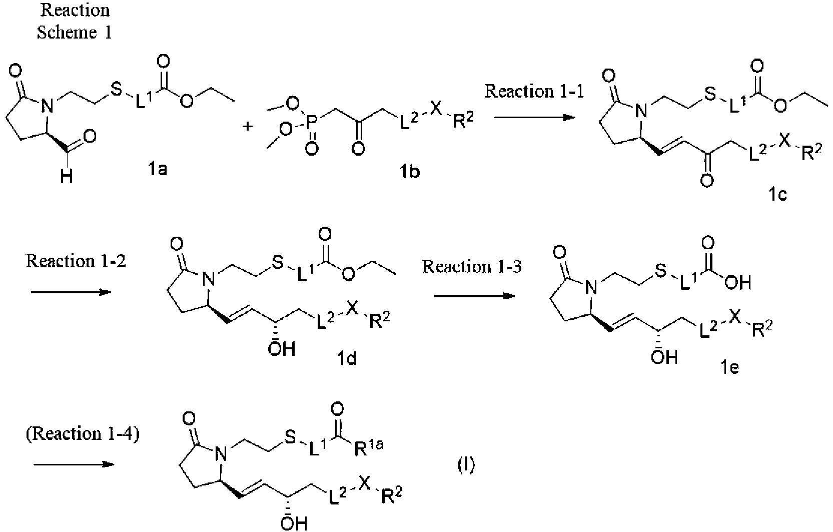

(In Reaction Scheme 1, R^{1a} represents an -NHCH₂CH₂SO₃H group, an - NHCH₂COOH group, or an -NHCH(COOH)CH₂CH₂COOH group, and the other symbols represent the same meaning as described above)
In Reaction Scheme 1, Reaction 1-1 is known, and is performed, for example, by reacting a compound represented by general formula 1a and a compound represented by general formula 1b in an organic solvent (for example, tetrahydrofuran (THF), dimethylformamide (DMF), dimethoxyethane (DME), dioxane, acetonitrile, ethanol, and dichloromethane), water, or a mixed solution thereof, in the presence of a base (for example, sodium hydride, sodium hydroxide, potassium hydroxide, potassium phosphate, potassium tert-butoxide, potassium carbonate, tertiary amine + lithium chloride, or the like) at a temperature of -20 to 70°C.

In Reaction Scheme 1, Reaction 1-2 is known, and is performed by reacting the compound represented by general formula 1c obtained in Reaction 1-1 in an organic solvent (for example, THF, DME, toluene, dichloromethane, diethyl ether, dioxane, or the like), in the presence or absence of cerium chloride, using a reducing agent (for example, sodium borohydride, zinc borohydride, or the like) at -20 to 50°C. When only one stereoisomer is selectively produced, the reaction is performed using an asymmetric reducing agent (for example, chlorodiisopinocamphenylborane or the like) or a combination of a chiral auxiliary and a reducing agent ((R)-2-methyl-CBS-oxazaborolidine and a borohydride/tetrahydrofuran complex or a borane dimethyl sulfide complex; (S)-(-)-binaphthol and lithium aluminum hydride; or the like) at a temperature of -100 to 50°C.

In Reaction Scheme 1, Reaction 1-3 is a deprotection reaction and is performed under the same conditions as the deprotection reaction described above.

In Reaction Scheme 1, Reaction 1-4 is an amidation reaction. The amidation reaction is known, and examples thereof include:
(1) a method using an acid halide,
(2) a method using a mixed acid anhydride, and
(3) a method using a condensing agent.

These methods are specifically described as follows:
(1) The method using an acid halide is performed, for example, by reacting a carboxylic acid with an acid halide-forming agent (oxalyl chloride, thionyl chloride, or the like) in an organic solvent (chloroform, dichloromethane, diethyl ether, THF, or the like) or in the absence of a solvent at -20°C to reflux temperature, and reacting the obtained acid halide with an amine in the presence of a base (pyridine, triethylamine, dimethylaniline, dimethylaminopyridine, diisopropylethylamine, or the like) in an organic solvent (chloroform, dichloromethane, diethyl ether, THF, or the like) at a temperature of 0 to 40°C. The method can also be performed by reacting the obtained acid halide with an amine in an organic solvent (dioxane, THF, or the like) using an aqueous alkali solution (sodium bicarbonate solution, sodium hydroxide solution, or the like) at 0 to 40°C.
(2) The method using a mixed acid anhydride is performed, for example, by reacting a carboxylic acid with an acid halide (pivaloyl chloride, tosyl chloride, mesyl chloride, or the like) or an acid derivative (ethyl chloroformate, isobutyl chloroformate, or the like) in an organic solvent (chloroform, dichloromethane, diethyl ether, THF, or the like) or in the absence of a solvent, in the presence of a base (pyridine, triethylamine, dimethylaniline, dimethylaminopyridine, diisopropylethylamine, or the like) at 0 to 40°C, and reacting the obtained mixed acid anhydride with an amine in an organic solvent (chloroform, dichloromethane, diethyl ether, THF, or the like) at 0 to 40°C.
(3) The method using a condensing agent is performed, for example, by reacting a carboxylic acid with an amine in an organic solvent (chloroform, dichloromethane, DMF, diethyl ether, THF, or the like) or in the absence of a solvent, in the presence or absence of a base (pyridine, triethylamine, dimethylaniline, dimethylaminopyridine, or the like), using a condensing agent (1,3-dicyclohexylcarbodiimide (DCC), 1-ethyl-3-[3-(dimethylamino)propyl]carbodiimide (EDC), 1,1'-carbonyldiimidazole (CDI), 2-chloro-1-methylpyridinium iodine, 1-propylphosphonic acid cyclic anhydride (1-propanephosphonic acid cyclic anhydride, PPA), or the like), O-(7-azabenzotriazol-1-yl)-N,N,N',N'-tetramethyluronium hexafluorophosphate (HATU), in the presence or absence of 1-hydroxybenztriazole (HOBt) at 0 to 40°C.

All of these reactions (1), (2), and (3) are desirably performed under an inert gas (argon, nitrogen, or the like) atmosphere under an anhydrous condition.

In Reaction Scheme 1, Reaction 1-4 can be omitted as appropriate depending on the compound to be obtained and the intermediate to be used.

Among the compounds represented by general formula (1c), a compound in which X is X¹ can also be produced by Reaction Scheme 2.

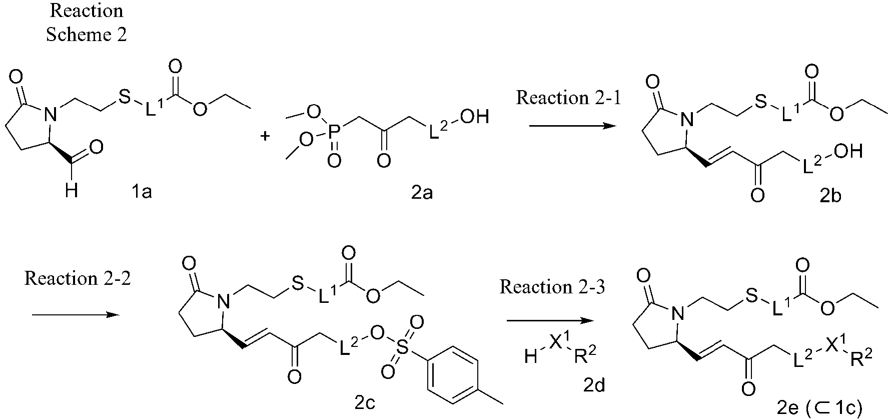

(In Reaction Scheme 2, all symbols represent the same meaning as described above)
In Reaction Scheme 2, Reaction 2-1 is known, and can be performed by being subjected to the same conditions as in Reaction 1-1 described above.

In Reaction Scheme 2, Reaction 2-2 is known, and can be performed by reacting p-toluenesulfonyl chloride in an organic solvent (for example, dichloromethane, diethyl ether, THF, acetonitrile, benzene, toluene) in the presence of a base (for example, pyridine, triethylamine, dimethylaniline, dimethylaminopyridine, diisopropylethylamine, or the like) at a temperature of 0 to 100°C.

In Reaction Scheme 2, Reaction 2-3 is known, and is performed, for example, by reacting in an organic solvent (dimethylformamide, dimethyl sulfoxide, chloroform, dichloromethane, diethyl ether, tetrahydrofuran, methyl t-butyl ether, or the like) in the presence of a hydroxide of an alkali metal (sodium hydroxide, potassium hydroxide, lithium hydroxide, or the like), a hydroxide of an alkaline earth metal (barium hydroxide, calcium hydroxide, or the like), a carbonate (sodium carbonate, potassium carbonate, or the like), an aqueous solution thereof, or a mixture thereof at 0 to 100°C.

In each reaction in the present specification, the compounds represented by general formula 1a, general formula 1b, and general formula 2a, which were used as starting materials, and the compound represented by general formula 2d are known, or can be easily produced by combining and using methods described in WO 2003007941 A (Patent Literature 1), Comprehensive Organic Transformations: A Guide to Functional Group Preparations, 3rd Edition (Richard C. Larock, John Wiley & Sons Inc, 2018), or the like, methods obtained by partially modifying known methods, or the like.

Among the compounds of the present disclosure, a compound having optical activity can be produced by using a starting material having optical activity or a reagent; optically resolving a racemic production intermediate, then leading to a compound of the present disclosure; or optically resolving a racemic compound of the present disclosure.

This optical resolution is known, and examples thereof include a method in which a salt, a complex, or the like is formed with another optically active compound, recrystallization is performed, and then a target compound is isolated, or directly separated using a chiral column or the like.

In each reaction in the present specification, a reaction involving heating can be performed using a water bath, an oil bath, a sand bath, or a microwave, as will be apparent to those skilled in the art.

In each reaction in the present specification, a solid-phase-supported reagent supported on a macromolecular polymer (for example, polystyrene, polyacrylamide, polypropylene, polyethylene glycol, or the like) may be appropriately used.

In each reaction in the present specification, the reaction product can be purified by ordinary purification means, for example, distillation under normal pressure or reduced pressure, high performance liquid chromatography using silica gel or magnesium silicate, thin layer chromatography, ion exchange resin, scavenger resin or column chromatography, or washing, recrystallization, or the like. The purification may be performed for each reaction or may be performed after completion of some reactions.

### [Toxicity]

Since the toxicity of the compound of the present disclosure is low, the compound of the present disclosure can be used safely.

### [Pharmaceutical composition]

Since the compound of the present disclosure has agonist activity for an EP4 receptor, the compound of the present disclosure may be useful for prevention and/or treatment of diseases related to an EP4 receptor, for example, immune diseases (autoimmune diseases such as amyotrophic lateral sclerosis (ALS), multiple sclerosis, Sjögren's syndrome, chronic rheumatoid arthritis, and systemic lupus erythematosus; rejection after organ transplantation; and the like), asthma, neuronal cell death, arthritis, lung injury, pulmonary fibrosis, pulmonary emphysema, bronchitis, chronic obstructive pulmonary disease, liver injury, acute hepatitis, nephritis (acute nephritis, chronic nephritis), renal failure, hypertension, myocardial ischemia, systemic inflammatory response syndrome, sepsis, hemophagocytic syndrome, macrophage activation syndrome, Still's disease, Kawasaki disease, burn injury, systemic granuloma, inflammatory bowel disease (ulcerative colitis, Crohn's disease, and the like), hypercytokinemia during dialysis, multiple organ failure, shock, and the like. The EP4 receptor is also related to a mucosa-protecting action, and the compound of the present disclosure may be useful for prevention and/or treatment of gastrointestinal ulcers such as gastric ulcers and duodenal ulcers and stomatitis.

The compound that selectively binds to EP4 has no pain generation that is considered to be caused by EP1, no uterine contraction action that is considered to be caused by EP3, and the like, and thus the compound of the present disclosure may be a drug having no side effect due to these pain generation and uterine contraction action.

In order to use the compound of the present disclosure for the purpose of preventing and/or treating the above diseases, the compound as an active ingredient is usually formulated together with a pharmaceutically acceptable carrier such as various additives or solvents, and then administered systemically or topically, orally or parenterally. Here, the pharmaceutically acceptable carrier means a substance other than the active ingredient, which is generally used for pharmaceutical preparations. It is preferable that the pharmaceutically acceptable carrier does not exhibit a pharmacological action at a dose of the preparation, is harmless, and does not interfere with the therapeutic effect of the active ingredient. The pharmaceutically acceptable carrier can also be used for the purpose of, for example, enhancing the usefulness of the active ingredient and the preparation, facilitating the formulation, stabilizing the quality, or improving the usability. Specifically, substances described in "Japanese Pharmaceutical Excipients Directory" published in 2021 by Yakuji Nippo, Limited (edited by International Pharmaceutical Excipients Council Japan) and the like may be appropriately selected according to the purpose.

The compound of the present disclosure may be administered to a mammal (preferably a human, and more preferably a human patient) in a pharmaceutically effective amount.

The dose of the compound of the present disclosure depends on age, body weight, symptom, desired therapeutic effect, route of administration, period of treatment, and the like, and thus inevitably varies. Generally, the compound of the present disclosure is administered orally in a range of 0.1 ng to 1000 mg per patient per dose, or parenterally in a range of 0.01 ng to 100 mg per patient per dose, or continuously administered intravenously.

Of course, as mentioned above, since the dose varies depending on various conditions, a dose smaller than the above dose may be sufficient, or administration beyond the range may be necessary.

An examples of the dosage form thereof include preparations for oral administration (e.g., tablets, capsules, granules, powders, oral solutions, syrups, oral jellies, and the like), preparations for oral cavity (e.g., oral tablets, oral sprays, oral semisolids, oral rinses, and the like), preparations for injection (e.g., injections, and the like), preparations for dialysis (e.g., dialysis agents, and the like), preparations for inhalation (e.g., inhalants, and the like), ophthalmic preparations (e.g., eye drops, eye ointments, and the like), otological preparations (e.g., ear drops, and the like), nasal preparations (e.g., nose drops, and the like), preparations for rectum (e.g., suppositories, semisolids for rectum, enteric preparations, and the like), vaginal preparations (e.g., vaginal tablets, vaginal suppositories, and the like), and preparations for skin (e.g., external solid agents, external liquid agents, sprays, ointments, creams, gels, patches, and the like).

### [Preparation for oral administration]

Examples of the preparation for oral administration include tablets, capsules, granules, powders, oral solutions, syrups, and oral jellies. Examples of the preparation for oral administration include rapidly disintegrating preparations in which the releasability of the active ingredient from the preparation is not particularly adjusted, and modified-release preparations such as enteric preparations and sustained-release preparations in which the releasability is adjusted according to the purpose by unique preparation design and manufacturing method. The enteric preparation refers to a preparation designed to release the active ingredient mainly in the small intestine without releasing the active ingredient in the stomach for the purpose of preventing degradation of the active ingredient in the stomach or reducing irritation of the active ingredient to the stomach, and can be usually produced by applying a coating using an acid-insoluble enteric base. The sustained-release preparation refers to a preparation in which the release rate, release time, and release site of the active ingredient from the preparation are adjusted for the purpose of reducing the frequency of administration or reducing side effects, and can be usually produced by using an appropriate sustained-release agent. Among preparations for oral administration, capsules, granules, tablets, and the like can be coated with an appropriate coating agent such as a saccharide, a sugar alcohol, or a polymer compound for the purpose of, for example, facilitating ingestion or preventing degradation of the active ingredient.

### (1) Tablet

The tablet is a solid preparation that is administered orally and have a certain shape, and examples thereof include those generally referred to as tablets such as uncoated tablets, film-coated tablets, dragees, multilayer tablets, and dry-coated tablets, as well as orally rapidly disintegrating tablets, chewable tablets, effervescent tablets, dispersing tablets, and dissolving tablets. When the uncoated tablet is produced, the following method (a), (b), or (c) is usually used:
(a) An additive such as an excipient, a binder, or a disintegrant is added to an active ingredient and mixed to homogenize the mixture, and the mixture is formed into particles by an appropriate method using water or a solution containing a binder, then a lubricant or the like is added to the mixture, the mixture is mixed, and compression molding is performed;
(b) An additive such as an excipient, a binder, or a disintegrant is added to an active ingredient and mixed to homogenize the mixture, and the mixture is directly compression molded, or the active ingredient, a lubricant, and the like are added to granules prepared in advance with the additive and mixed to homogenize the mixture, and then compression molding is performed;
(c) An additive such as an excipient or a binder is added to an active ingredient and mixed to homogenize the mixture, and the mixture wetted with a solvent is poured into a certain mold and molded, and then dried by an appropriate method.

The film-coated tablet can be usually produced by thinly coating an uncoated tablet with an appropriate coating agent such as a polymer compound. The dragee can be usually produced by coating an uncoated tablet with a coating agent containing a saccharide or a sugar alcohol. The multilayer tablet can be produced by laminating powder particles having different compositions in layers by an appropriate method and compression-molding the powder particles. The dry-coated tablet can be produced by covering an inner core tablet with an outer layer having a different composition. The tablet can also be made into an enteric tablet or a sustained-release tablet using a known appropriate method. The orally rapidly disintegrating tablet, the chewable tablet, the effervescent tablet, the dispersing tablet, and the dissolving tablet are those in which unique functions are imparted to the tablet by appropriate selection of additives, and can be produced according to the method for producing the tablet. The orally rapidly disintegrating tablet refers to a tablet that can be taken by being rapidly dissolved or disintegrated in the oral cavity; the chewable tablet refers to a tablet that is taken by being chewed; the effervescent tablet refers to a tablet that dissolves or disperses while rapidly foaming in water; the dispersing tablet refers to a tablet that is taken by being dispersed in water; and the dissolving tablet refers to a tablet that is taken by being dissolved in water. The effervescent tablet can be produced by using an appropriate acidic substance, a carbonate, a hydrogen carbonate, or the like as an additive.

### (2) Capsule

The capsule is a preparation filled in a capsule or encapsulated with a capsule base, and examples thereof include hard capsules and soft capsules. The hard capsule can be produced by adding an additive such as an excipient to an active ingredient and mixing to homogenize the mixture, or forming into particles or a molded product by an appropriate method, and filling the mixture or the particles or molded product into a capsule as it is/they are or by lightly molding the mixture or the particles. The soft capsule can be produced by encapsulating a mixture of an active ingredient and an additive in a certain shape with an appropriate capsule base such as gelatin having increased plasticity by adding glycerin, D-sorbitol, or the like. The capsule can also be made into an enteric capsule or a sustained-release capsule using a known appropriate method, and a colorant, a preservative, or the like can also be added to the capsule base.

### (3) Granule

The granule is a preparation granulated into particles, and examples thereof include effervescent granules in addition to those generally referred to as granules. When the granule is produced, the following method (a), (b), or (c) is usually used:
(a) An excipient, a binder, a disintegrant, or other additives are added to a powdery active ingredient to homogenize the mixture, and then the mixture is formed into particles by an appropriate method;
(b) An additive such as an excipient is added to an active ingredient prepared into particles in advance and mixed to homogenize the mixture;
(c) An additive such as an excipient is added to an active ingredient prepared into particles in advance and mixed, and the mixture is formed into particles by an appropriate method.

The granule can also be coated as necessary, and can also be made into an enteric granule or a sustained-release granule using a known appropriate method. An effervescent granule can be produced by using an appropriate acidic substance, a carbonate, a hydrogen carbonate, or the like as an additive. The effervescent granule refers to a granule that dissolves or disperses while rapidly foaming in water. The granule can also be made into a fine granule by adjusting the particle size.

### (4) Powder

The powder is a powdery preparation, and can be usually produced by adding an excipient or other additives to an active ingredient and mixing them to homogenize the mixture.

### (5) Oral solution

The oral solution is a liquid or flowable viscous gel-like preparation, and examples thereof include those generally referred to as oral solutions, as well as elixirs, suspensions, emulsions, and limonades. The oral solution can be usually produced by adding an additive and purified water to an active ingredient, mixing them, homogeneously dissolving or emulsifying or suspending them, and filtering them as necessary. The elixir refers to a clear liquid oral solution containing sweet and aromatic ethanol, and can be usually produced by adding ethanol, purified water, a flavoring agent, white sugar, and other saccharides, or a sweetener to a solid active ingredient or a leachate thereof and dissolving them to form a clear liquid by filtration or other methods. The suspension refers to an oral solution in which an active ingredient is finely and homogeneously suspended, and can be usually produced by adding a suspending agent or other additives and purified water or oil to a solid active ingredient, suspending the mixture by an appropriate method, and homogenizing the entire mixture. The emulsion refers to an oral solution in which an active ingredient is finely and homogeneously emulsified, and can be usually produced by adding an emulsifier and purified water to a liquid active ingredient, emulsifying the mixture by an appropriate method, and homogenizing the entire mixture. The limonade refers to a clear liquid oral solution having a sweet taste and a sour taste.

### (6) Syrup

The syrup is a viscous liquid or solid preparation containing saccharides or a sweetener, and examples thereof include syrup preparations. The syrup can be usually produced by adding an active ingredient to a solution of white sugar, other saccharides, or a sweetener, or a single syrup, dissolving, mixing, suspending, or emulsifying the mixed liquid, and if necessary, boiling the mixed liquid, and then filtering the mixed liquid at the time of heating. The syrup preparation refers to a granular or powdery preparation that becomes a syrup when water is added, and may also be referred to as a dry syrup. The syrup preparation can be usually produced using saccharides or a sweetener as an additive according to the method for producing the granule or powder.

### (7) Oral Jelly

The oral jelly is a molded gel-like preparation having no fluidity, and can be usually produced by adding an additive and a polymer gel base to an active ingredient, mixing them, gelatinizing them by an appropriate method, and molding them into a certain shape.

### [Preparation for Injection]

### (1) Injection

The injection is a solid sterile preparation that is administered subcutaneously or intramuscularly or directly administered to a body tissue such as blood vessel, or an organ, is used as a solution, a suspension, or an emulsion, or by being dissolved or suspended during use, and examples thereof include those generally referred to as injections, as well as lyophilized injections, powder injections, pre-filled syringes, cartridge preparations, infusions, implant injections, and long acting injections. When the injection is produced, the following method (a) or (b) is usually used:
(a) A solution obtained by dissolving, suspending, or emulsifying an active ingredient as it is or a mixture obtained by adding an additive to the active ingredient in water for injection, another aqueous solvent, a non-aqueous solvent, or the like to homogenize the mixture is filled into a container for injection, and the container is sealed and sterilized;
(b) A solution obtained by dissolving, suspending, or emulsifying an active ingredient as it is or a mixture obtained by adding an additive to the active ingredient in water for injection, another aqueous solvent, a non-aqueous solvent, or the like to homogenize the mixture is aseptically filtered, or aseptically prepared to homogenize the mixture is filled into a container for injection, and the container is sealed.

The lyophilized injection can be usually produced by dissolving an active ingredient as it is or the active ingredient and an additive such as an excipient in water for injection, aseptically filtering the solution, filling the solution into a container for injection, and then lyophilizing the solution, or lyophilizing the solution in a dedicated container, and then filling the solution into a direct container. The powder injection can be usually produced by treating a powder by aseptic filtration, then performing crystallization, or adding a sterilized additive to the powder, and filling the powder into a container for injection. The pre-filled syringe can be usually produced by preparing a solution, a suspension, or an emulsion with an active ingredient as it is or with the active ingredient and an additive and filling the solution, the suspension, or the emulsion into a syringe barrel. The cartridge preparation refers to an injection used by placing a cartridge filled with a liquid medicine in a dedicated syringe, and the cartridge filled with a liquid medicine can be usually produced by preparing a solution, a suspension, or an emulsion with an active ingredient as it is or with the active ingredient and an additive and filling the solution, the suspension, or the emulsion into the cartridge. The infusion refers to an injection usually having a volume of 100 mL or more administered intravenously. The implant injection refers to a solid or gel-like injection that is applied subcutaneously, intramuscularly, or the like using an implantation tool or by surgery for the purpose of releasing an active ingredient over a long period of time. The implant injection can be usually produced by using a biodegradable polymer compound and forming it into a pellet, microsphere, or gel form. The long acting injection refers to an injection that is applied intramuscularly or the like for the purpose of releasing an active ingredient over a long period of time, and can be usually produced by dissolving or suspending the active ingredient in a vegetable oil or the like, or preparing a suspension of a microsphere using a biodegradable polymer compound.

The compound of the present disclosure may be administered in combination with other drugs for:
1) complementing and/or enhancing the preventive and/or therapeutic effect of the compound,
2) improving kinetics and absorption of the compound, reducing the dose of the compound, and/or
3) reducing side effects of the compound.

The combination agent of the compound of the present disclosure and other drugs may be administered in the form of a compounded agent in which both components are compounded in one preparation, or may be administered in the form of separate preparations. This administration in the form of separate preparations includes simultaneous administration and administration at time intervals. In addition, for administration at time intervals, the compound of the present disclosure may be administered first and another drug may be administered later, or another drug may be administered first and the compound of the present disclosure may be administered later. The respective administration methods may be the same or different.

The disease on which a preventive and/or therapeutic effect is exerted by the above combination agent is not particularly limited as long as it is a disease for which the preventive and/or therapeutic effect of the compound of the present disclosure is complemented and/or enhanced.

Examples of other drugs for complementing and/or enhancing the preventive and/or therapeutic effect of the compound represented by general formula (I) on inflammatory bowel disease include 5-aminosalicylic acid preparations, steroids, immunomodulators, immunosuppressants, anti-TNF-α antibodies, anti-α4β7 integrin antibodies, and JAK inhibitors.

Examples of the 5-aminosalicylic acid preparation include salazosulfapyridine and mesalazine.

Examples of the steroid include prednisolone, betamethasone, budesonide, and cortisol.

Examples of the immunomodulator include azathioprine and 6-mercaptopurine.

Examples of the immunosuppressant include tacrolimus and cyclosporine.

Examples of the anti-TNF-α antibody include infliximab, adalimumab, and golimumab.

Examples of the anti-α4β7 integrin antibody include vedolizumab.

Examples of the JAK inhibitor include tofacitinib.

The weight ratio of the compound represented by general formula (I) to other drugs is not particularly limited.

In addition, any two or more of other drugs may be administered in combination.

In addition, other drugs that complement and/or enhance the preventive and/or therapeutic effect of the compound of the present disclosure include not only those that have been found to date but also those that will be found in the future based on the mechanism described above.

Unless defined otherwise, all technical and scientific terms, and abbreviations used herein have the same meaning as commonly understood by those skilled in the art to which the present invention belongs.

In addition, in the present specification, all contents of all Patent Literatures and Non Patent Literatures or references explicitly cited may be cited herein as part of the present specification.

In one aspect, the present disclosure provides the following embodiments.
[1] A compound represented by general formula (I): 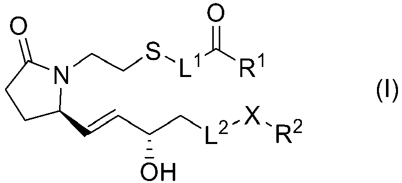 wherein
   R¹ represents (1) an -OH group, (2) an -NHCH₂CH₂SO₃H group, (3) an - NHCH₂COOH group, or (4) an -NHCH(COOH)CH₂CH₂COOH group;
   L¹ represents (1) a thiazole ring or (2) C1-4 alkylene;
   L² represents (1) C3-5 alkylene or (2) a benzene ring;
   X represents (1) -CO-, (2) -NR^{X}-, or (3) a sulfur atom;
   R^{X} represents (1) a hydrogen atom or (2) a C1-4 alkyl group;
   R² represents (1) a methyl group, (2) a benzene ring, (3) a 5- to 10-membered heterocyclic ring, (4) a -COR²⁻¹ group, (5) a -CO-(benzene ring) group, (6) a -CO-(5- to 10-membered heterocyclic ring) group, (7) an -NH-(benzene ring) group, or (8) an -OH group;
   when R² represents a benzene ring, a 5- to 10-membered heterocyclic ring, a -CO-(benzene ring) group, a -CO-(5- to 10-membered heterocyclic ring) group, or an -NH-(benzene ring) group, the benzene ring or the 5- to 10-membered heterocyclic ring may be substituted with 1 to 5 R²⁻²s;
   R²⁻¹ represents (1) a C1-4 alkyl group that may be substituted with a C1-4 alkoxy group or (2) an -NH-(C1-4 alkyl) group; and
   R²⁻² represents (1) a halogen atom, (2) a C1-4 alkyl group, (3) a C1-4 haloalkyl group, (4) a C1-4 alkoxy group, (5) a C1-4 haloalkoxy group, or (6) a benzene ring; or a pharmaceutically acceptable salt thereof.
[2] The compound according to [1] or a pharmaceutically acceptable salt thereof, wherein L¹ is a thiazole ring, and the thiazole ring is represented by: 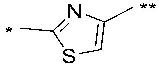 wherein * represents a bonding site with a sulfur atom, and ** represents a bonding site with -COR¹.
[3] The compound according to [1] or [2], or a pharmaceutically acceptable salt thereof, wherein the 5- to 10-membered heterocyclic ring in R² is a heterocyclic ring selected from the group consisting of (1) a pyridine ring, (2) a pyrazine ring, (3) a pyrimidine ring, (4) an imidazole ring, (5) a thiazole ring, (6) a triazole ring, (7) a tetrazole ring, (8) a furan ring, (9) a quinoline ring, and (10) a pyrazole ring.
[4] The compound according to [1] or [2], or a pharmaceutically acceptable salt thereof, which is represented by general formula (I-1): 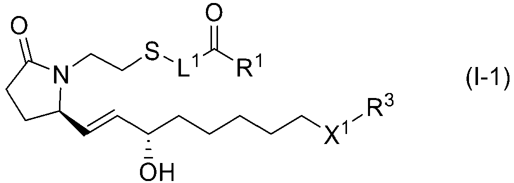
   wherein R³ represents (1) a benzene ring, (2) a pyridine ring, (3) a pyrazine ring, (4) a pyrimidine ring, (5) an imidazole ring, (6) a thiazole ring, (7) a triazole ring, (8) a tetrazole ring, (9) a furan ring, (10) a quinoline ring, or (11) a pyrazole ring;
   R³ may be substituted with 1 to 5 R³⁻¹s;
   R³⁻¹ represents (1) a halogen atom, (2) a C1-4 alkyl group, (3) a C1-4 haloalkyl group, (4) a C1-4 alkoxy group, (5) a C1-4 haloalkoxy group, or (6) a benzene ring;
   X¹ represents (1) -NR^{X}- or (2) a sulfur atom; and
   the other symbols represent the same meaning as the symbols described in [1] or [2].
[5] The compound according to [4] or a pharmaceutically acceptable salt thereof, which is represented by general formula (I-2): 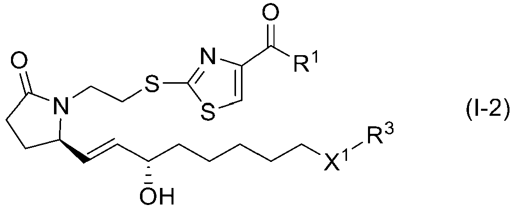 wherein all symbols represent the same meaning as the symbols described in [4].
[6] The compound according to any one of [1] to [5], or a pharmaceutically acceptable salt thereof, which is represented by general formula (I-3): 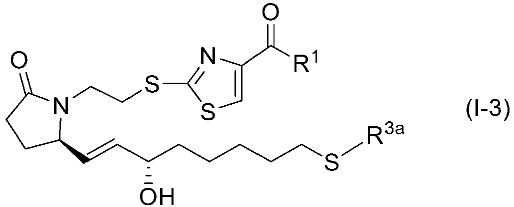 wherein R^{3a} represents (1) a pyridine ring or (2) a pyrazine ring, R^{3a} may be substituted with 1 to 3 R³⁻¹s, and the other symbols represent the same meaning as described in any one of [1] to [5].
[7] The compound according to [1] or [2], or a pharmaceutically acceptable salt thereof, which is represented by general formula (I-4): 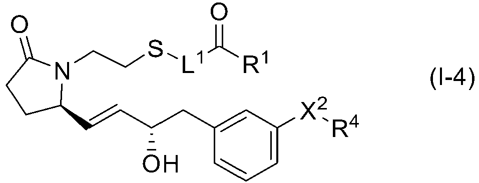
   wherein R⁴ represents (1) a -COR⁴⁻¹ group, (2) a -CO-(benzene ring) group, (3) a - CO-(pyridine ring) group, (4) a -CO-(pyrazine ring) group, (5) a -CO-(furan ring) group, (6) an -NH-(benzene ring) group, or (7) an -OH group;
   when R⁴ represents a -CO-(benzene ring) group, a -CO-(pyridine ring) group, a -CO-(pyrazine ring) group, a -CO-(furan ring) group, or an -NH-(benzene ring) group, the benzene ring, the pyridine ring, the pyrazine ring, or the furan ring may be substituted with 1 to 5 R⁴⁻²s;
   R⁴⁻¹ represents (1) a C1-4 alkyl group that may be substituted with a C1-4 alkoxy group or (2) an -NH-(C1-4 alkyl) group;
   R⁴⁻² represents (1) a halogen atom, (2) a C1-4 alkyl group, (3) a C1-4 haloalkyl group, (4) a C1-4 alkoxy group, (5) a C1-4 haloalkoxy group, or (6) a benzene ring;
   X² represents (1) -CO- or (2) -NR^{X}-; and
   the other symbols represent the same meaning as the symbols described in [1] or [2].
[8] The compound according to [1], wherein the compound represented by general formula (I) is a compound selected from the group consisting of:
   (1) 2-[(2-{(2R)-2-[(1E,3S)-3-hydroxy-8-(2-pyridinylthio)-1-octen-1-yl]-5-oxo-1-pyrrolidinyl}ethyl)thio]-1,3-thiazole-4-carboxylic acid;
   (2) 2-[(2-{(2R)-2-[(1E,3S)-3-hydroxy-8-(2-pyrazinylthio)-1-octen-1-yl]-5-oxo-1-pyrrolidinyl}ethyl)thio]-1,3-thiazole-4-carboxylic acid;
   (3) 2-({2-[(2R)-2-{(1E,3S)-3-hydroxy-8-[methyl(phenyl)amino]-1-octen-1-yl}-S-oxo-1-pyrrolidinyl]ethyl}thio)-1,3-thiazole-4-carboxylic acid;
   (4) 2-({2-[(2R)-2-{(1E,3S)-3-hydroxy-8-[(3-methoxy-2-pyrazinyl)thio]-1-octen-1-yl}-5-oxo-1-pyrrolidinyl]ethyl}thio)-1 ,3-thiazole-4-carboxylic acid;
   (5) 2-[(2-{(2R)-2-[(1E,3S)-3-hydroxy-9-(2-pyridinylthio)-1-nonen-1-yl]-5-oxo-1-pyrrolidinyl}ethyl)thio]-1,3-thiazole-4-carboxylic acid;
   (6) 4-({2-[(2R)-2-{(1E,3S)-4-[3-(benzoylamino)phenyl]-3-hydroxy-1-buten-1-yl}-S-oxo-1-pyrrolidinyl]ethyl}thio)butanoic acid;
   (7) 2-[({2-[(2-{(2R)-2-[(1E,3S)-3-hydroxy-8-(2-pyridinylthio)-1-octen-1-yl]-5-oxo-1-pyrrolidinyl}ethyl)thio]-1,3-thiazol-4-yl}carbonyl)amino]ethanesulfonic acid;
   (8) 2-({[2-({2-[(2R)-2-{(1E,3S)-3-hydroxy-8-[(3-methoxy-2-pyrazinyl)thio]-1-octen-1-yl}-5-oxo-1-pyrrolidinyl]ethyl}thio)-1,3-thiazol-4-yl]carbonyl}amino)ethanesulfonic acid;
      and
   (9) 2-{[4-({2-[(2R)-2-{(1E,3S)-4-[3-(benzoylamino)phenyl]-3-hydroxy-1-buten-1-yl}-5-oxo-1-pyrrolidinyl]ethyl}thio)butanoyl]amino } ethanesulfonic acid; or a pharmaceutically acceptable salt thereof.
[9] A pharmaceutical composition comprising the compound according to any one of [1] to [8], or a pharmaceutically acceptable salt thereof, as an active ingredient, and further comprising a pharmaceutically acceptable carrier.
[10] The pharmaceutical composition according to [8], which is an EP4 agonist.
[11] The pharmaceutical composition according to [8], which is a therapeutic and/or preventive agent for a disease related to an EP4 receptor.
[12] The pharmaceutical composition according to [11], wherein the disease related to an EP4 receptor is immune disease, asthma, neuronal cell death, arthritis, lung injury, pulmonary fibrosis, pulmonary emphysema, bronchitis, chronic obstructive pulmonary disease, liver injury, acute hepatitis, nephritis, renal failure, hypertension, myocardial ischemia, systemic inflammatory response syndrome, sepsis, hemophagocytic syndrome, macrophage activation syndrome, Still's disease, Kawasaki disease, burn injury, systemic granuloma, inflammatory bowel disease, hypercytokinemia during dialysis, multiple organ failure, and/or shock.
[13] The pharmaceutical composition according to [11], wherein the disease related to an EP4 receptor is inflammatory bowel disease.
[14] The pharmaceutical composition according to [11], wherein the disease related to an EP4 receptor is inflammatory bowel disease, and the inflammatory bowel disease is ulcerative colitis or Crohn's disease.
[15] A therapeutic and/or preventive agent for a disease related to an EP4 receptor, the therapeutic and/or preventive agent containing the compound according to any one of [1] to [8] or a pharmaceutically acceptable salt thereof.
[16] A method for preventing and/or treating a disease related to an EP4 receptor, the method including administering the compound according to any one of [1] to [8] or a pharmaceutically acceptable salt thereof, or a pharmaceutical composition containing the compound according to any one of [1] to [8] or a pharmaceutically acceptable salt thereof as an active ingredient and further a pharmaceutically acceptable carrier to a patient in need of prevention and/or treatment of a disease related to an EP4 receptor.
[17] A compound according to any one of [1] to [8] or a pharmaceutically acceptable salt thereof, for use in prevention and/or treatment of a disease related to an EP4 receptor.
[18] Use of the compound according to any one of [1] to [8] or a pharmaceutically acceptable salt thereof, for the manufacture of a preventive and/or therapeutic agent for a disease related to an EP4 receptor.

The compounds of the present disclosure are described in detail below based on the following examples; however, the compounds of the present disclosure are not limited to these examples.

### [Synthesis Examples]

The solvent in parentheses shown in the portion of separation by chromatography and TLC indicates an eluting solvent or developing solvent used, and the proportion represents a volume ratio.

The solvent in parentheses shown in the portion of NMR indicates a solvent used for measurement.

The compound names used in the present specification are named using ACD/Name (registered trademark) or Chemdraw Ultra (version 12.0, manufactured by Cambridge Soft Corporation), which is a computer program that generally performs naming according to the IUPAC rules; or named according to the IUPAC nomenclature.

LC-MS/ELSD was performed under the following conditions.

### Conditions;

Column: YMC Triart C₁₈ (particle size: 1.9 x 10⁻⁶ m; column length: 30 x 2.0 mm I.D.); flow rate: 1.0 mL/min; column temperature: 30°C; mobile phase (A): 0.1% trifluoroacetic acid aqueous solution; mobile phase (B): 0.1% trifluoroacetic acid-acetonitrile solution; gradient (ratio of mobile phase (A): mobile phase (B) is described): [0 min] 95 : 5; [0.1 min] 95 : 5; [1.2 min] 5 : 95; [1.6 min] 5 : 95; detector: UV (PDA), ELSD, MS.

The HPLC retention time indicates a retention time under the conditions described in the LC-MS/ELSD if not stated otherwise. The description in parentheses shown in the portion of HPLC retention time indicates measurement conditions.

### Reference Example 1: Dimethyl (7-hydroxy-2-oxoheptyl)phosphonate

To a tetrahydrofuran (THF) (120 mL) solution of dimethyl methylphosphonate (CAS No.: 756-79-6, 18 g), a 1.59 M n-hexane solution of n-butyllithium (CAS No.: 109-72-8, 100 mL) was added dropwise at -78°C, and the mixture was stirred at -78°C for 1 hour. A THF (20 mL) solution of epsilon-caprolactone (CAS No.: 502-44-3,8.3 g) was added dropwise at - 78°C, and the mixture was stirred for 1 hour. A saturated aqueous ammonium chloride solution was added to the reaction mixture, and the mixture was extracted with ethyl acetate. The extract was washed with saturated saline, dried over sodium sulfate, and then filtered. The filtrate was concentrated under reduced pressure to give a title compound (16.7 g, purity 79%) having the following physical property value.

TLC: Rf 0.31 (ethyl acetate : methanol = 19 : 1).

### Reference Example 2: Ethyl 2-[(2-{(2R)-2-[(1E)-8-hydroxy-3-oxo-1-octen-1-yl]-5-oxo-1-pyrrolidinyl}ethyl)thio]-1,3-thiazole-4-carboxylate

To a THF (210 mL) solution of ethyl 2-({2-[(2R)-2-(formyl)-5-oxo-1-pyrrolidinyl]ethyl}thio)-1,3-thiazole-4-carboxylate (CAS No.: 1355026-69-5, 10 g), the compound (7.2 g) produced in Reference Example 1, lithium chloride (3.9 g), and triethylamine (11 mL) were added, and the mixture was stirred at room temperature for 4 hours. Water was added to the reaction mixture, and the mixture was extracted with ethyl acetate. The extract was washed with saturated saline, dried over sodium sulfate, and then filtered, and the filtrate was concentrated under reduced pressure. The obtained residue was purified by silica gel column chromatography (hexane : ethyl acetate : methanol = 50 : 50 : 0 → 0 : 100 : 0 → 0 : 90 : 10) to give a title compound (9.1 g) having the following physical property value.

TLC: Rf 0.2 (ethyl acetate).

### Reference Example 3: Ethyl 2-[(2-{(2R)-2-[(1E)-8-{[(4-methylphenyl)sulfonyl]oxy}-3-oxo-1-octen-1-yl]-5-oxo-1-pyrrolidinyl}ethyl)thio]-1,3-thiazole-4-carboxylate

To a dichloromethane (56 mL) solution of the compound (8 g) produced in Reference Example 2, trimethylamine hydrochloride (694 mg), triethylamine (7.6 mL), and p-toluenesulfonyl chloride (4.5 g) were added, and the mixture was stirred at room temperature through the night. Water was added to the reaction mixture, and the mixture was extracted with ethyl acetate. The extract was washed with saturated saline, dried over sodium sulfate, and then filtered, and the filtrate was concentrated under reduced pressure. The obtained residue was purified by silica gel column chromatography (hexane : ethyl acetate = 20 : 80 → 0 : 100) to give a title compound (9.3 g) having the following physical property value.

HPLC retention time (min): 1.17.

### Reference Example 4: Ethyl 2-[(2-{(2R)-2-[(1E)-3-hydroxy-8-{[(4-methylphenyl)sulfonyl]oxy}-1-octen-1-yl]-5-oxo-1-pyrrolidinyl}ethyl)thio]-1,3-thiazole-4-carboxylate

To a THF (18 mL) solution of the compound (2.5 g) produced in Reference Example 3, a 1 M toluene solution (5 mL) of (R)-(+)-2-methyl-CBS-oxazaborolidine and N,N-diethylaniline borane (0.86 mL) were added at -15°C, and the mixture was stirred at -15°C for 4 hours. Water and a 1 N aqueous hydrochloric acid solution were added to the reaction mixture, and the mixture was extracted with ethyl acetate. The extract was washed with saturated saline, dried over sodium sulfate, and then filtered, and the filtrate was concentrated under reduced pressure. The obtained residue was purified by silica gel column chromatography (ethyl acetate : methanol = 100 : 0 → 90 : 10) to give a title compound (1.86 g) having the following physical property value as a diastereomer mixture.

TLC: Rf 0.2 (ethyl acetate).

### Reference Example 5: Ethyl 2-[(2-{(2R)-2-[(1E)-3-hydroxy-8-(2-pyridinylthio)-1-octen-1-yl]-5-oxo-1-pyrrolidinyl}ethyl)thio]-1,3-thiazole-4-carboxylate

To an acetonitrile (2.4 mL) solution of the compound (120 mg) produced in Reference Example 4, potassium carbonate (42 mg), sodium iodide (45 mg), and 2-mercaptopyridine (CAS No.: 2637-34-5, 34 mg) were added, and the mixture was stirred at 60°C for 6 hours. Water was added to the reaction mixture, and the mixture was extracted with ethyl acetate. The extract was dried over sodium sulfate, and then filtered, and the filtrate was concentrated under reduced pressure. The obtained residue was purified by silica gel column chromatography (ethyl acetate : methanol = 100 : 0 → 90 : 10) to give a title compound (87 mg) having the following physical property value as a diastereomer mixture.

HPLC retention time (min): 0.94.

### Example 1: 2-[(2-{(2R)-2-[(1E,3S)-3-Hydroxy-8-(2-pyridinylthio)-1-octen-1-yl]-5-oxo-1-pyrrolidinyl}ethyl)thio]-1,3-thiazole-4-carboxylic acid

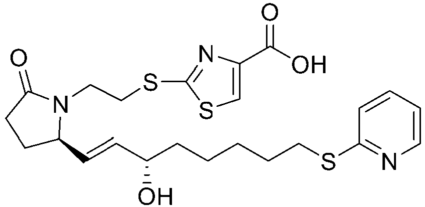

To a dimethoxyethane (0.8 mL)-methanol (0.8 mL) solution of the compound (86 mg) produced in Reference Example 5, a 2 N aqueous sodium hydroxide solution (0.34 mL) was added, and the reaction mixture was stirred at room temperature for 1 hour. The reaction mixture was purified by reverse phase column chromatography to separate two diastereomers, thereby obtaining a low-polar substance (70 mg).
HPLC retention time (min): 0.83;
¹H-NMR (CD₃OD): δ 8.35, 8.23, 7.57-7.62, 7.25, 7.06, 5.74, 5.45-5.52, 4.29-4.35, 3.97-4.03, 3.79, 3.32-3.51, 3.24-3.29, 3.07-3.14, 2.18-2.43, 1.58-1.81, 1.24-1.50.

### Examples 1-1 to 1-16:

A procedure for a purpose similar to that for Reference Example 5 → Example 1 was carried out by using a corresponding thiol compound in place of 2-mercaptopyridine to give the following example compounds.

### Example 1-1: 2-[(2-{(2R)-2-[(1E,3S)-3-Hydroxy-8-(2-pyrazinylthio)-1-octen-1-yl]-5-oxo-1-pyrrolidinyl}ethyl)thio]-1,3-thiazole-4-carboxylic acid

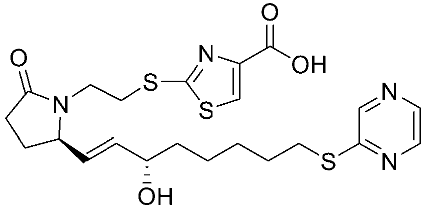

HPLC retention time (min): 0.923;
¹H-NMR (DMSO-d₆): δ 8.56, 8.47, 8.30, 5.66, 5.38, 4.10-4.23, 3.88, 3.67, 3.06-3.23, 2.55, 2.34-2.47, 2.07-2.32, 1.51-1.68, 1.15-1.39.

### Example 1-2: 2-({2-[(2R)-2-{(1E,3S)-3-Hydroxy-8-[(6-methyl-2-pyrazinyl)thio]-1-octen-1-yl}-5-oxo-1-pyrrolidinyllethyl}thio)-1,3-thiazole-4-carboxylic acid

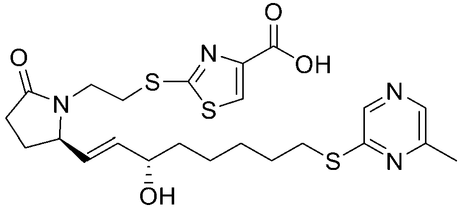

HPLC retention time (min): 0.956;
¹H-NMR (DMSO-d₆): δ 8.34, 8.26, 8.19, 5.67, 5.39, 4.06-4.19, 3.88, 3.61-3.73, 3.08-3.18, 2.41-2.47, 2.07-2.30, 1.48-1.68, 1.15-1.40.

### Example 1-3: 2-({2-[(2R)-2-{(1E,3S)-3-Hydroxy-8-[(6-methyl-2-pyridinyl)thio]-1-octen-1-yl}-5-oxo-1-pyrrolidinyl]ethyl}thio)-1,3-thiazole-4-carboxylic acid

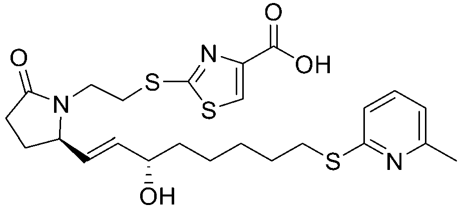

HPLC retention time (min): 0.827;
¹H-NMR (CD₃OD): δ 8.22, 7.49, 7.04, 6.92, 5.74, 5.48, 4.28-4.35, 4.01, 3.78, 3.32-3.51, 3.24-3.30, 3.08, 2.45, 2.15-2.43, 1.60-1.81, 1.23-1.52.

### Example 1-4: 2-({2-[(2R)-2-{(1E,3S)-8-[(3-Chloro-2-pyridinyl)thio]-3-hydroxy-1-octen-1-yl}-5-oxo-1-pyrrolidinyllethyl}thio)-1,3-thiazole-4-carboxylic acid

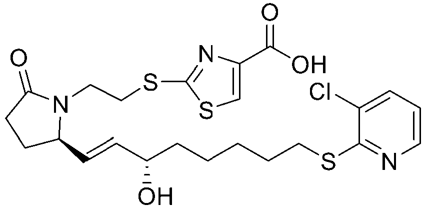

HPLC retention time (min): 1.064;
¹H-NMR (DMSO-d₆): δ 8.43, 8.31, 7.83, 7.16, 5.66, 5.39, 4.72, 3.99-4.23, 3.88, 3.67, 3.07-3.22, 2.04-2.31, 1.46-1.74, 1.17-1.42.

### Example 1-5: 2-({2-[(2R)-2-{(1E,3S)-3-Hydroxy-8-[(4-methyl-2-pyridinyl)thio]-1-octen-1-yl}-5-oxo-1-pyrrolidinyllethyl}thio)-1,3-thiazole-4-carboxylic acid

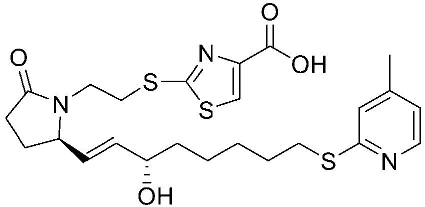

HPLC retention time (min): 0.834;
¹H-NMR (DMSO-d₆): δ 8.26, 7.09, 6.91, 5.65, 5.31-5.47, 4.62-4.87, 4.15, 3.87, 3.54-3.76, 2.98-3.21, 2.03-2.28, 1.50-1.68, 1.14-1.39.

### Example 1-6: 2-({2-[(2R)-2-{(1E,3S)-3-Hydroxy-8-[(3-methyl-2-pyridinyl)thio]-1-octen-1-yl}-5-oxo-1-pyrrolidinyl]ethyl}thio)-1,3-thiazole-4-carboxylic acid

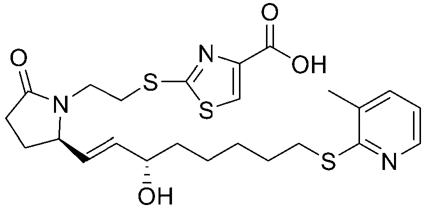

HPLC retention time (min): 1.011;
¹H-NMR (DMSO-d₆): δ 8.24-8.34, 7.48, 7.02, 5.66, 5.39, 4.72, 4.09-4.20, 3.79-3.94, 3.63-3.72, 3.08-3.19, 2.05-2.31, 1.48-1.70, 1.16-1.43.

### Example 1-7: 2-({2-[(2R)-2-{(1E,3S)-3-Hydroxy-8-[(3-methoxy-2-pyridinyl)thio]-1-octen-1-yl}-5-oxo-1-pyrrolidinyllethyl}thio)-1,3-thiazole-4-carboxylic acid

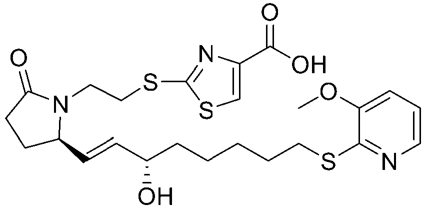

HPLC retention time (min): 0.984;
¹H-NMR (DMSO-d₆): δ 12.50-13.46, 8.33, 8.03, 7.24, 7.08, 5.65, 5.38, 4.70, 4.10-4.20, 4.05-4.24, 3.85-3.92, 3.79-3.84, 3.63-3.73, 3.36-3.41, 2.98-3.23, 2.06-2.34, 1.44-1.70, 1.16-1.42.

### Example 1-8: 2-({2-[(2R)-2-{(1E,3S)-8-[(5,6-Difluoro-2-pyridinyl)thio]-3-hydroxy-1-octen-1-yl}-5-oxo-1-pyrrolidinyl]ethyl}thio)-1,3-thiazole-4-carboxylic acid

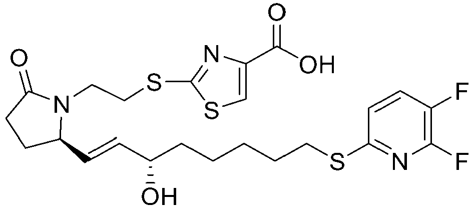

HPLC retention time (min): 1.049;
¹H-NMR (DMSO-d₆): δ 8.33, 7.82, 6.94, 5.66, 5.39, 4.72, 4.08-4.20, 3.89, 3.67, 3.03-3.24, 2.06-2.32, 1.53-1.69, 1.16-1.39.

### Example 1-9: 2-({2-[(2R)-2-{(1E,3S)-8-[(3-Fluoro-2-pyridinyl)thio]-3-hydroxy-1-octen-1-yl}-5-oxo-1-pyrrolidinyl]ethyl}thio)-1,3-thiazole-4-carboxylic acid

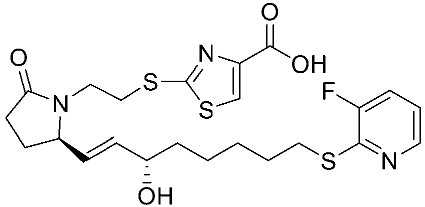

HPLC retention time (min): 1.018;
¹H-NMR (DMSO-d₆): δ 8.33, 7.53-7.66, 7.20, 5.65, 5.39, 4.71, 4.00-4.23, 3.88, 3.67, 3.08-3.21, 2.04-2.32, 1.47-1.75, 1.16-1.42.

### Example 1-10: 2-[(2-{(2R)-2-[(1E,3S)-3-Hydroxy-8-(3-pyridinylthio)-1-octen-1-yl]-5-oxo-1-pyrrolidinyl}ethyl)thio]-1,3-thiazole-4-carboxylic acid

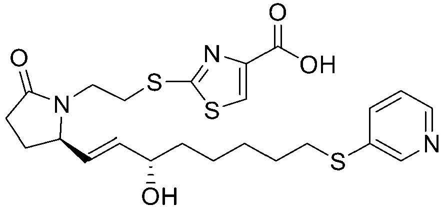

HPLC retention time (min): 0.829;
¹H-NMR (DMSO-d₆): δ 8.50, 8.29-8.41, 7.76, 7.33, 5.65, 5.38, 4.71, 4.01-4.24, 3.83-3.91, 3.61-3.72, 3.08-3.17, 2.94-3.01, 2.06-2.34, 1.45-1.69, 1.12-1.39.

### Example 1-11: 2-[(2-{(2R)-2-[(1E,3S)-3-Hydroxy-8-(1H-1,2,4-triazol-3-ylthio)-1-octen-1-yl]-5-oxo-1-pyrrolidinyl}ethyl)thio]-1,3-thiazole-4-carboxylic acid

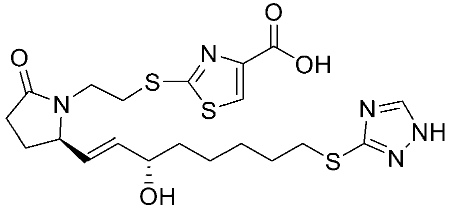

HPLC retention time (min): 0.813;
¹H-NMR (DMSO-d₆): δ 8.31, 5.65, 5.38, 4.71, 4.09-4.20, 3.87, 3.62-3.73, 3.00-3.08, 2.06-2.32, 1.47-1.69, 1.20-1.41.

### Example 1-12: 2-[(2-{(2R)-2-[(1E,3S)-3-Hydroxy-8-(1H-pyrazolyl-3-ylthio)-1-octen-1-yl]-5-oxo-1-pyrrolidinyl}ethyl)thio]-1,3-thiazole-4-carboxylic acid

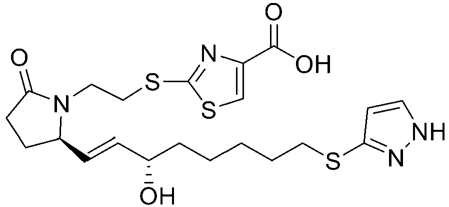

HPLC retention time (min): 0.882;
¹H-NMR (DMSO-d₆): δ 8.31, 7.66, 6.25, 5.65, 5.38, 4.71, 3.99-4.27, 3.83-3.92, 3.66, 3.09-3.22, 2.82, 2.06-2.32, 1.58-1.69, 1.41-1.56, 1.10-1.36.

### Example 1-13: 2-({2-[(2R)-2-{(1E,3S)-3-Hydroxy-8-[(1-phenyl-1H-tetrazol-5-yl)thio]-1-octen-1-yl}-5-oxo-1-pyrrolidinyl]ethyl}thio)-1,3-thiazole-4-carboxylic acid

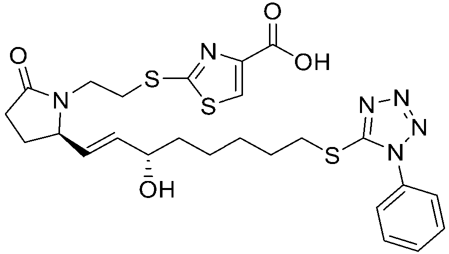

HPLC retention time (min): 1.008;
¹H-NMR (DMSO-d₆): δ 8.30, 7.59-7.71, 5.65, 5.38, 4.72, 4.00-4.24, 3.87, 3.62-3.71, 3.11-3.19, 2.05-2.30, 1.58-1.80, 1.16-1.41.

### Example 1-14: 2-[(2-{(2R)-2-[(1E,3S)-3-Hydroxy-8-(8-quinolinylthio)-1-octen-1-yl]-5-oxo-1-pyrrolidinyl}ethyl)thio]-1,3-thiazole-4-carboxylic acid

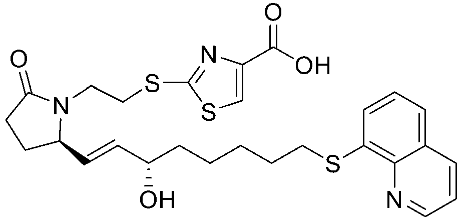

HPLC retention time (min): 0.969;
¹H-NMR (DMSO-d₆): δ 8.88, 8.28-8.38, 7.70, 7.49-7.60, 5.66, 5.38, 4.73, 4.00-4.24, 3.90, 3.62-3.71, 3.09-3.19, 2.97-3.04, 2.04-2.30, 1.52-1.77, 1.21-1.48.

### Example 1-15: 2-[(2-{(2R)-2-[(1E,3S)-3-Hydroxy-8-(1H-imidazol-2-ylthio)-1-octen-1-yl]-5-oxo-1-pyrrolidinyl}ethyl)thio]-1,3-thiazole-4-carboxylic acid

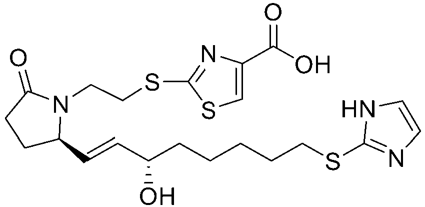

HPLC retention time (min): 0.721;
¹H-NMR (DMSO-d₆): δ 8.33, 7.03, 5.63, 5.38, 4.55-4.90, 4.09-4.18, 3.81-3.89, 3.66, 3.07-3.14, 2.93, 2.06-2.30, 1.55-1.70, 1.39-1.55, 1.21-1.35.

### Example 1-16: 2-[(2-{(2R)-2-[(1E,3S)-3-Hydroxy-8-(1,3-thiazol-2-ylthio)-1-octen-1-yl]-5-oxo-1-pyrrolidinyl}ethyl)thio]-1,3-thiazole-4-carboxylic acid

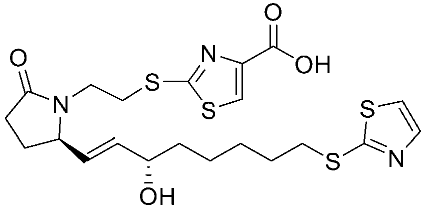

HPLC retention time (min): 0.947;
¹H-NMR (DMSO-d₆): δ 12.77-13.47, 8.35, 7.71, 7.64, 5.65, 5.39, 4.71, 4.11-4.19, 3.83-3.92, 3.65-3.72, 3.08-3.21, 2.53-2.60, 2.05-2.31, 1.54-1.73, 1.15-1.40.

### Example 2: 2-({2-[(2R)-2-{(1E,3S)-3-Hydroxy-8-[methyl(phenyl)amino]-1-octen-1-yl}-5-oxo-1-pyrrolidinyl]ethyl}thio)-1,3-thiazole-4-carboxylic acid

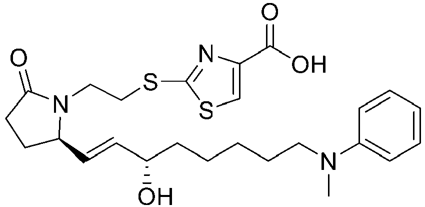

A procedure for a purpose similar to that for Reference Example 5 → Example 1 was carried out by using N-methylaniline in place of 2-mercaptopyridine to give a title compound having the following physical property values.
TLC: Rf 0.69 (chloroform : methanol = 4 : 1);
¹H-NMR (CD₃OD): δ 8.14, 7.14-7.21, 6.75-6.80, 6.68, 5.67-5.77, 5.39-5.51, 4.26-4.34, 3.98, 3.72-3.81, 3.39-3.48, 2.91, 2.16-2.40, 1.69-1.80, 1.35-1.55, 1.21-1.35.

### Reference Example 6: Ethyl 2-[(2-{(2R)-2-[(1E,3S)-8-(acetylthio)-3-{[dimethyl(2-methyl-2-proyanyl)silyl]oxy}-1-octen-1-yl]-5-oxo-1-pyrrolidinyl}ethyl)thio]-1,3-thiazolyl-4-carboxylate

To a DMF (2.6 mL) solution of the compound (133 mg) produced in Reference Example 4, imidazole (23 mg) and t-butyldimethylsilyl chloride (37 mg) were added, and the mixture was stirred at room temperature for 2 hours. Water was added to the reaction mixture, and the mixture was extracted with ethyl acetate. The extract was washed with saturated saline, dried over sodium sulfate, and then filtered, and the filtrate was concentrated under reduced pressure. To a dimethylacetamide (1.3 mL) solution of the obtained residue, potassium thioacetate (CAS No.: 10387-40-3, 28 mg) was added, and the mixture was stirred at 60°C for 30 minutes. Water was added to the reaction mixture, and the mixture was extracted with ethyl acetate. The extract was washed with saturated saline, dried over sodium sulfate, and then filtered, and the filtrate was concentrated under reduced pressure. The obtained residue was purified by silica gel column chromatography (hexane : ethyl acetate = 50 : 50) to give a title compound (91 mg) having the following physical property value.

HPLC retention time (min): 1.491.

### Example 3: 2-({2-[(2R)-2-{(1E,3S)-3-Hydroxy-8-[(3-methoxy-2-pyrazinyl)thio]-1-octen-1-yl}-5-oxo-1-pyrrolidinyl]ethyl}thio)-1,3-thiazole-4-carboxylic acid

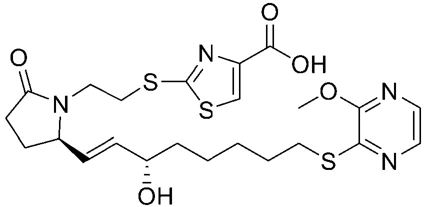

To an ethanol (0.9 mL) solution of the compound (30 mg) produced in Reference Example 6, triphenylphosphine (14 mg), potassium carbonate (10 mg), and 2-bromo-3-methoxypyrazine (CAS No.: 1209905-41-8, 10 mg) were added, and the mixture was stirred at 60°C all day and night. Saturated saline was added to the reaction mixture, and the mixture was extracted with ethyl acetate. The extract was dried over sodium sulfate, and then filtered, and the filtrate was concentrated under reduced pressure. To a THF (0.2 mL) solution of the obtained residue, a 1 N tetrabutylammonium fluoride (0.2 mL) solution was added, and the mixture was stirred at room temperature all day and night. To the reaction mixture, methanol (0.5 mL) and a 2 N aqueous sodium hydroxide solution (0.2 mL) were added, and the mixture was stirred at room temperature for 2 hours. The reaction mixture was purified by reverse phase column chromatography to separate two diastereomers, thereby obtaining a low-polar substance (17 mg).
HPLC retention time (min): 1.01;
¹H-NMR (CDCl₃): δ 8.09, 7.94, 7.75, 5.76, 5.58, 4.10-4.17, 4.02, 3.78, 3.41-3.55, 3.29-3.36, 3.13-3.18, 2.19-2.57, 1.74, 1.34-1.54.

### Example 3-1: 2-({2-[(2R)-2-{(1E,3S)-3-Hydroxy-8-[(6-methoxy-2-pyrazinyl)thio]-1-octen-1-yl}-5-oxo-1-pyrrolidinyl]ethyl}thio)-1,3-thiazole-4-carboxylic acid

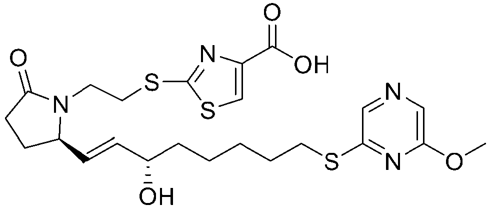

A procedure for a purpose similar to that for Example 3 was carried out by using 2-bromo-6-methoxypyrazine in place of 2-bromo-3-methoxypyrazine to give a title compound having the following physical property values.
HPLC retention time (min): 0.476;
¹H-NMR (DMSO-d₆): δ 8.24-8.35, 8.11, 7.95, 5.66, 5.38, 4.06-4.23, 3.86-3.94, 3.50-3.77, 2.97-3.24, 2.65, 2.06-2.33, 1.51-1.69, 1.14-1.40.

### Reference Example 7: Dimethyl (6-hydroxy-2-oxohexyl)phosphonate

A procedure for a purpose similar to that for Reference Example 1 was carried out by using delta-valerolactone (CAS No.: 542-28-9) in place of epsilon-caprolactone to give a title compound having the following physical property value.

TLC: Rf 0.3 (ethyl acetate : methanol = 20 : 1).

### Examples 4-1 to 4-4:

A procedure for a purpose similar to that for Reference Example 2 → Reference Example 3 → Reference Example 4 → Reference Example 5 → Example 1 was carried out by using the compound produced in Reference Example 7 in place of the compound produced in Reference Example 1 and using a corresponding thiol compound in place of 2-mercaptopyridine to give the following example compounds.

### Example 4-1: 2-[(2-{(2R)-2-[(1E,3S)-3-Hydroxy-7-(2-pyridinylthio)-1-hepten-1-yl]-5-oxo-1-pyrrolidinyl}ethyl)thio]-1,3-thiazole-4-carboxylic acid

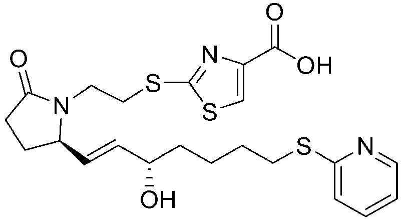

TLC: Rf 0.29 (methanol : chloroform : acetic acid = 1 : 8 : 0.1);
¹H-NMR (CD₃OD): δ 8.52, 8.25, 8.15, 7.76, 7.58-7.46, 5.74, 5.50, 4.36-4.25, 4.10-3.97, 3.87-3.73, 3.46, 3.40-3.20, 2.48-2.13, 1.87-1.64, 1.63-1.37.

### Example 4-2: 2-({2-[(2R)-2-{(1E,3S)-3-Hydroxy-7-[(5-methyl-2-pyrazinyl)thio]-1-hepten-1-yl}-5-oxo-1-pyrrolidinyl]ethyl}thio)-1,3-thiazole-4-carboxylic acid

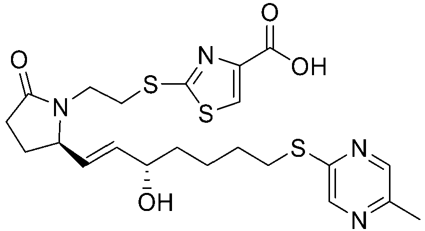

HPLC retention time (min): 0.91;
¹H-NMR (CDCl₃): δ 8.38, 8.27, 8.01, 5.81, 5.54, 4.08-4.19, 3.63-3.73, 3.35-3.50, 3.27, 3.04-3.15, 2.49, 2.28-2.47, 2.19-2.27, 1.57-1.82, 1.33-1.56, 0.96.

### Example 4-3: 2-[(2-{(2R)-2-[(1E,3S)-7-{[6-(difluoromethoxy)-2-pyridinyl]thio}-3-hydroxy-1-hepten-1-yl]-5-oxo-1-pyrrolidinyl}ethyl)thio]-1,3-thiazole-4-carboxylic acid

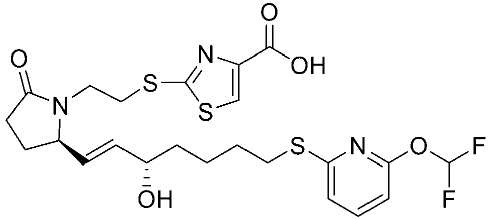

HPLC retention time (min): 1.04;
¹H-NMR (CD₃OD): δ 7.92, 7.28-7.75, 7.02, 6.58-6.61, 5.73, 5.43-5.52, 4.58, 4.29-4.42, 3.95-4.08, 3.73-3.88, 3.40-3.52, 3.33-3.36, 2.10-2.42, 1.58-1.78, 1.37-1.55, 1.29.

### Example 4-4: 2-({2-[(2R)-2-{(1E,3S)-3-Hydroxy-7-[(5-methyl-2-pyridinyl)thio]-1-hepten-1-yl}-5-oxo-1-pyrrolidinyl]ethyl}thio)-1,3-thiazole-4-carboxylic acid

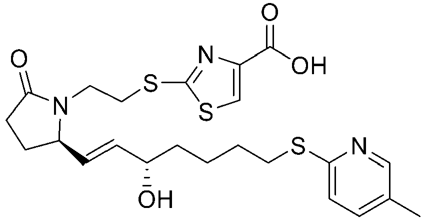

HPLC retention time (min): 0.894;
¹H-NMR (CD₃OD): δ 8.23, 8.21, 7.46, 7.16, 5.73, 5.48, 4.28-4.34, 4.01, 3.80, 3.39-3.54, 3.32-3.37, 3.07, 2.17-2.43, 1.60-1.81, 1.35-1.54.

### Reference Example 8: Dimethyl (8-{[dimethyl(2-methyl-2-propanyl)silyl]oxy}-2-oxooctyl)phosphonate

A procedure for a purpose similar to that for Example 1 was carried out by using methyl 7-{[dimethyl(2-methyl-2-propanyl)silyl]oxy}heptanoate (CAS No.: 181274-06-6) in place of epsilon-caprolactone to give a title compound having the following physical property value.

TLC: Rf 0.4 (ethyl acetate).

### Reference Example 9: Ethyl 2[(2-{(2R)-2-[(1E)-9-{[dimethyl(2-methyl-2-propanyl)silyl]oxy}-3-hydroxy-1-nonen-1-yl]-5-oxo-1-pyrrolidinyl}ethyl)thio]-1,3-thiazole-4-carboxylate

A procedure for a purpose similar to that for Reference Example 2 → Reference Example 4 was carried out by using the compound produced in Reference Example 8 in place of the compound produced in Reference Example 1 to give a title compound having the following physical property value.

HPLC retention time (min): 1.413.

### Reference Example 10: Ethyl 2-[(2-{(2R)-2-[(1E)-3-acetoxy-9-hydroxy-1-nonen-1-yl]-5-oxo-1-pyrrolidinyl} ethyl)thio]-1,3-thiazole-4-carboxylate

To a pyridine (3.6 mL) solution of the compound (360 mg) produced in Reference Example 9, N,N-dimethyl-4-aminopyridine (7.7 mg) and acetic anhydride (77 mg) were added, and the reaction mixture was stirred at room temperature all day and night. A 1 N aqueous hydrochloric acid solution was added to the reaction mixture, and the mixture was extracted with ethyl acetate. The extract was dried over sodium sulfate, and then filtered, and the filtrate was concentrated under reduced pressure. The obtained crude product was dissolved in ethanol (3.6 mL), and p-toluenesulfonic acid monohydrate (17 mg) was added thereto at 0°C, then the reaction mixture was stirred at room temperature for 1 hour. The reaction mixture was purified by silica gel column chromatography (hexane : ethyl acetate = 50 : 50 → 0 : 100) to give a title compound (265 mg) having the following physical property value.

HPLC retention time (min): 1.091.

### Example 5: 2-[(2-{(2R)-2-[(1E,3S)-3-Hydroxy-9-(2-pyridinylthio)-1-nonen-1-yl]-5-oxo-1-pyrrolidinyl}ethyl)thio]-1,3-thiazole-4-carboxylic acid

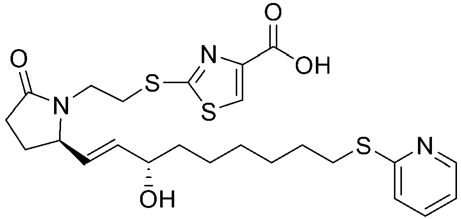

A procedure for a purpose similar to that for Reference Example 3 → Reference Example 5 → Example 1 was carried out by using the compound produced in Reference Example 10 in place of the compound produced in Reference Example 2 to give a title compound having the following physical property values.
HPLC retention time (min): 0.985;
¹H-NMR (DMSO-d₆): δ 8.41, 8.30, 7.59-7.64, 7.26, 7.08, 5.65, 5.38, 4.71, 4.09-4.20, 3.78-3.94, 3.67, 3.06-3.17, 2.05-2.31, 1.53-1.67, 1.13-1.38.

### Example 6: 4-[(2-{(2R)-2-[(1E,3S)-3-Hydroxy-8-(2-pyridinylthio)-1-octen-1-yl]-5-oxo-1-pyrrolidinyl}ethyl)thio]butanoic acid

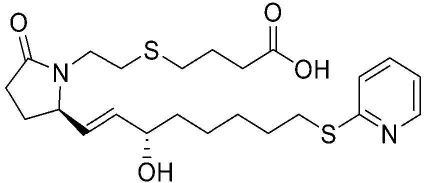

A procedure for a purpose similar to that for Reference Example 2 → Reference Example 3 → Reference Example 4 → Reference Example 5 → Example 1 was carried out by using butyl 4-({2-[(2R)-2-formyl-5-oxo-1-pyrrolidinyl]ethyl}thio)butanoic acid (CAS No.: 1365393-21-0) in place of ethyl 2-({2-[(2R)-2-(formyl)-5-oxo-1-pyrrolidinyl]ethyl}thio)-1,3-thiazole-4-carboxylate to give a title compound having the following physical property values.
HPLC retention time (min): 0.84;
¹H-NMR (CDCl₃): δ 8.43-8.46, 7.51, 7.21, 7.01, 5.76, 5.54, 4.10-4.21, 3.53-3.65, 3.12-3.23, 2.64-2.72, 2.34-2.62, 2.19-2.30, 1.86-1.94, 1.70-1.78, 1.40-1.60.

### Example 7: 4-[(2-{(2R)-2-[(1E,3S)-3-Hydroxy-7-(2-pyridinylthio)-1-hepten-1-yl]-5-oxo-1-pyrrolidinyl}ethyl)thio]butanoic acid

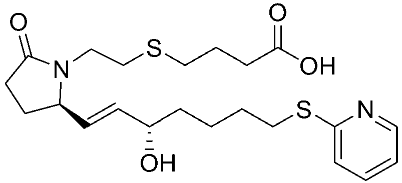

A procedure for a purpose similar to that for Reference Example 2 → Reference Example 3 → Reference Example 4 → Reference Example 5 → Example 1 was carried out by using butyl 4-({2-[(2R)-2-formyl-5-oxo-1-pyrrolidinyl]ethyl}thio)butanoic acid (CAS No.: 1365393-21-0) in place of ethyl 2-({2-[(2R)-2-(formyl)-5-oxo-1-pyrrolidinyl]ethyl}thio)-1,3-thiazole-4-carboxylate and using the compound produced in Reference Example 7 in place of the compound produced in Reference Example 1 to give a title compound having the following physical properties.
HPLC retention time (min): 0.817;
¹H-NMR (CDCl₃): δ 8.45, 7.51, 7.21, 7.02, 5.75, 5.53, 4.09-4.22, 3.66, 3.07-3.20, 2.70, 2.34-2.63, 2.20-2.29, 1.85-2.02, 1.71-1.81, 1.42-1.65.

### Reference Example 11: Methyl [3-(2-{[dimethyl(2-methyl-2-propanyl)silyl]oxy}ethyl)phenyl]acetate

To a DMF (10 mL) solution of methyl 2-(3-(hydroxyethyl)phenyl)acetate (CAS No.: 1260900-49-9, 1 g), imidazole (490 mg) and t-butyldimethylsilyl chloride (1.05 g) were added, and the mixture was stirred at room temperature for 7 hours. Water was added to the reaction mixture, and the mixture was extracted with ethyl acetate. The extract was washed with saturated saline, dried over sodium sulfate, and then filtered, and the filtrate was concentrated under reduced pressure. The obtained residue was purified by silica gel column chromatography (hexane : ethyl acetate = 100 : 0 → 10 : 90) to give a title compound (1.5 g) having the following physical property value.

TLC: Rf 0.5 (hexane : ethyl acetate = 90 : 10).

### Reference Example 12: Dimethyl {3-[3-(2-{[dimethyl(2-methyl-2-propanyl)silyl]oxy}ethyl)phenyl]-2-oxopropyl}phosphonate

A procedure for a purpose similar to that for Reference Example 1 was carried out by using the compound (1 g) produced in Reference Example 11 in place of epsilon-caprolactone to give a title compound (1 g) having the following physical property value.

TLC: Rf 0.43 (hexane : ethyl acetate = 33 : 67);

### Reference Example 13: 4-({2-[(2R)-2-{(1E,3S)-3-Hydroxy-4-[3-(2-hydroxyethyl)phenyl]-1-buten-1-yl}-5-oxo-1-pyrrolidinyl]ethyl}thio)butanoic acid

A procedure for a purpose similar to that for Reference Example 2 → Reference Example 4 → Reference Example 10 → Example 1 was carried out by using butyl 4-({2-[(2R)-2-formyl-5-oxo-1-pyrrolidinyl]ethyl}thio)butanoic acid in place of ethyl 2-({2-[(2R)-2-(formyl)-5-oxo-1-pyrrolidinyl]ethyl}thio)-1,3-thiazole-4-carboxylate and using the compound produced in Reference Example 12 in place of the compound produced in Reference Example 1 to give a title compound having the following physical properties.
HPLC retention time (min): 0.824;
¹H-NMR (CDCl₃): δ 7.27-7.31, 7.06-7.14, 5.81, 5.58, 4.36-4.47, 4.05-4.17, 3.87, 3.46-3.57, 3.10, 2.83-2.91, 2.73-2.82, 2.60-2.69, 2.31-2.58, 2.18-2.28, 1.87, 1.67-1.79, 1.13-1.34, 0.73-0.94.

### Reference Example 14: 3-{2-[Methoxy(methyl)amino]-2-oxoethyl}-N-phenylbenzamide

To an acetonitrile (35 mL) solution of [3-(phenylcarbamoyl)phenyl]acetic acid (CAS No.: 50961-58-5, 1.1 g), EDCI-HCL (1 g), N,O-dimethylhydroxylamine hydrochloride (526 mg) and triethylamine (1.3 mL) were added, and the mixture was stirred at room temperature for 2 hours. A saturated aqueous ammonium chloride solution was added to the reaction mixture, and the mixture was extracted with ethyl acetate. The extract was dried over sodium sulfate, and then filtered, and the filtrate was concentrated under reduced pressure. The obtained residue was purified by silica gel column chromatography (hexane : ethyl acetate = 80 : 20 → 34 : 66) to give a title compound (750 mg) having the following physical property value.

HPLC retention time (min): 0.974.

### Reference Example 15: Dimethyl {2-oxo-3-[3-(phenylcarbamoyl)phenyl]propyl}phosphonate

A procedure for a purpose similar to that for Reference Example 1 was carried out by using the compound produced in Reference Example 14 in place of the compound produced in Reference Example 1 to give a title compound having the following physical property value.

HPLC retention time (min): 0.923.

### Example 8: 4-({2-[(2R)-2-{(1E,3S)-3-Hydroxy-4-[3-(phenylcarbamoyl)phenyl]-1-buten-1-yl}-5-oxo-1-pyrrolidinyl]ethyl}thio)butanoic acid

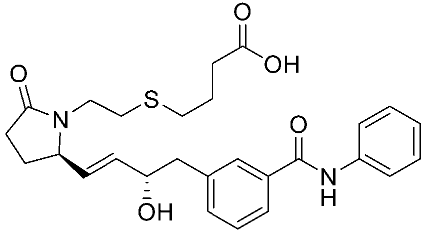

A procedure for a purpose similar to that for Reference Example 2 → Reference Example 4 → Example 1 was carried out by using butyl 4-({2-[(2R)-2-formyl-5-oxo-1-pyrrolidinyl]ethyl}thio)butanoic acid in place of ethyl 2-({2-[(2R)-2-(formyl)-5-oxo-1-pyrrolidinyl]ethyl}thio)-1,3-thiazole-4-carboxylate and using the compound produced in Reference Example 15 in place of the compound produced in Reference Example 1 to give a title compound having the following physical property values.
HPLC retention time (min): 0.939;
¹H-NMR (CD₃OD): δ 7.73-7.85, 7.66-7.72, 7.40-7.49, 7.33-7.40, 7.12-7.17, 5.74-5.83, 5.38-5.46, 4.90-5.07, 4.38-4.45, 4.13-4.22, 4.10, 3.43-3.65, 3.32-3.41, 2.85-3.07, 2.70-2.82, 2.44-2.67, 2.17-2.41, 2.00, 1.76-1.87, 1.58-1.74, 1.21-1.36, 0.93.

### Reference Example 16: Dimethyl [3-(3-nitrophenyl)-2-oxopropyl]phosphonate

A procedure for a purpose similar to that for Reference Example 1 was carried out by using N-methoxy-N-methyl-2-(3-nitrophenyl)acetamide (CAS No.: 1036397-64-4) in place of epsilon-caprolactone to give a title compound having the following physical property value.

TLC: Rf 0.39 (ethyl acetate).

### Reference Example 17: Butyl 4-[(2-{(2R)-2-[(1E)-3-hydroxy-4-(3-nitrophenyl)-1-buten-1-yl]-5-oxo-1-pyrrolidinyl}ethyl)thio]butanoate

A procedure for a purpose similar to that for Reference Example 2 → Reference Example 4 was carried out by using butyl 4-({2-[(2R)-2-formyl-5-oxo-1-pyrrolidinyl]ethyl}thio)butanoic acid in place of ethyl 2-({2-[(2R)-2-(formyl)-5-oxo-1-pyrrolidinyl]ethyl}thio)-1,3-thiazole-4-carboxylate and using the compound produced in Reference Example 16 in place of the compound produced in Reference Example 1 to give a title compound having the following physical property value.

HPLC retention time (min): 1.137.

### Reference Example 18: Butyl 4-[(2-{(2R)-2-[(1E)-3-acetoxy-4-(3-nitrophenyl)-1-buten-1-yl]-5-oxo-1-pyrrolidinyl}ethyl)thio]butanoate

To a dichloromethane (2.7 mL) solution of the compound (260 mg) produced in Reference Example 17, acetyl chloride (86 mg), triethylamine (0.23 mL), and N,N-dimethyl-4-aminopyridine (6.6 mg) were added, and the mixture was stirred at room temperature for 3 hours. A saturated aqueous sodium bicarbonate solution was added to the reaction mixture, and the mixture was extracted with ethyl acetate. The extract was washed with saturated saline, dried over sodium sulfate, and then filtered, and the filtrate was concentrated under reduced pressure. The obtained residue was purified by silica gel column chromatography (hexane : ethyl acetate = 20 : 80) to give a title compound (200 mg) having the following physical property value.

TLC: Rf 0.33 (hexane : ethyl acetate = 20 : 80).

### Reference Example 19: Butyl 4-[(2-{(2R)-2-[(1E)-3-acetoxy-4-(3-aminophenyl)-1-buten-1-yl]-5-oxo-1-pyrrolidinyl}ethyl)thio]butanoate

To a methanol (11 mL) solution of the compound (278 mg) produced in Reference Example 18, water (1 mL), THF (2.1 mL), ammonium chloride (500 mg), and zinc powder (611 mg) were added, and the mixture was stirred at 65°C for 15 hours. The reaction mixture was filtered, water was added to the filtrate, and the mixture was extracted with ethyl acetate. The extract was washed with saturated saline, dried over sodium sulfate, and then filtered, and the filtrate was concentrated under reduced pressure. The obtained residue was purified by silica gel column chromatography (hexane : ethyl acetate : methanol = 80 : 20 : 0 → 0 : 100 : 0 → 0 : 90 : 10). The obtained crude product was purified by reverse phase chromatography to give a title compound (32 mg) having the following physical property value.

TLC: Rf 0.22 (hexane : ethyl acetate = 20 : 80).

### Example 9: 4-[(2-{(2R)-2-[(1E,3S)-3-Hydroxy-4-{3-[(5-methyl-2-furoyl)amino]phenyl}-1-buten-1-yl]-5-oxo-1-pyrrolidinyl}ethyl)thio]butanoic acid

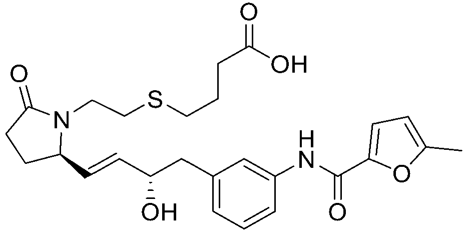

To a DMF (0.2 mL) solution of the compound (25 mg) produced in Reference Example 19, 5-methyl-2-furancarboxylic acid (CAS No.: 1917-13-2, 9.6 mg), triethylamine (0.014 mL), and HATU (25 mg) were added, and the mixture was stirred at room temperature for 2 hours. To the reaction solution, a 2 N aqueous sodium hydroxide solution (0.13 mL) was added, and the mixture was stirred at room temperature all day and night. To the reaction mixture, 2 N aqueous hydrochloric acid solution (0.25 mL) and dimethyl sulfoxide (0.1 mL) were added. The reaction mixture was purified by reverse phase column chromatography to separate two diastereomers, thereby obtaining a low-polar substance (16 mg).
HPLC retention time (min): 0.908;
¹H-NMR (DMSO-d₆): δ 9.91, 7.54-7.61, 7.19-7.27, 6.91, 6.31, 5.64-5.78, 5.31, 5.02, 4.17-4.27, 4.04, 2.74-2.83, 2.58-2.72, 2.42-2.47, 2.36-2.41, 2.07-2.29, 1.69, 1.41-1.62.

### Example 10: 4-({2-[(2R)-2-{(1E,3S)-4-[3-(Benzoylamino)phenyl]-3-hydroxy-1-buten-1-yl}-5-oxo-1-pyrrolidinyl]ethyl}thio)butanoic acid

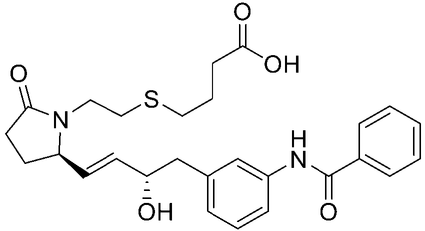

A procedure for a purpose similar to that for Example 9 was carried out by using benzoic acid in place of 5-methyl-2-furancarboxylic acid to give a title compound having the following physical property values.
HPLC retention time (min): 0.929;
¹H-NMR (DMSO-d₆): δ 11.92-12.33, 10.17, 7.93-7.97, 7.50-7.66, 7.24, 6.94, 5.69, 5.32, 5.02, 4.23, 4.00-4.07, 2.74-2.83, 2.60-2.74, 2.37-2.48, 2.07-2.33, 1.52-1.76.

### Examples 10-1 to 10-4:

A procedure for a purpose similar to that for Reference Example 17 → Reference Example 18 → Reference Example 19 → Example 9 was carried out by using ethyl 2-({2-[(2R)-2-(formyl)-5-oxo-1-pyrrolidinyl]ethyl}thio)-1,3-thiazole-4-carboxylate in place of butyl 4-({2-[(2R)-2-formyl-5-oxo-1-pyrrolidinyl]ethyl}thio)butanoic acid and using a corresponding carboxylic acid compound in place of 5-methyl-2-furancarboxylic acid to give the following example compounds.

### Example 10-1: 2-[(2-{(2R)-2-[(1E,3S)-3-Hydroxy-4-{3-[(methoxyacetyl)amino]phenyl}-1-buten-1-yl]-5-oxo-1-pyrrolidinyl}ethyl)thiol-1,3-thiazole-4-carboxylic acid

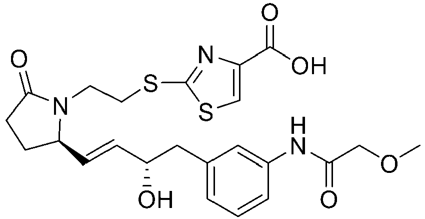

HPLC retention time (min): 0.812;
¹H-NMR (CD₃OD): δ 8.21, 7.42-7.47, 7.22, 6.93, 5.69, 5.29-5.37, 4.27-4.35, 4.15-4.26, 3.98-4.04, 3.70-3.77, 3.52-3.63, 3.43-3.46, 2.82-2.95, 2.65, 2.11-2.36, 1.84-1.89, 1.62-1.72.

### Example 10-2: 2-[(2-{(2R)-2-[(1E,3S)-3-Hydroxy-4-{3-[(2-pyridinylcarbonyl)amino]phenyl}-1-buten-1-yl]-5-oxo-1-pyrrolidinyl}ethyl)thio]-1,3-thiazole-4-carboxylic acid

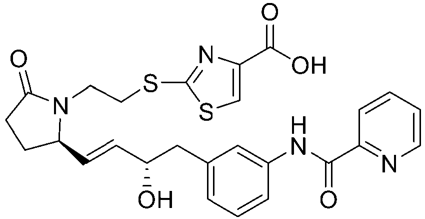

HPLC retention time (min): 0.903;
¹H-NMR (CD₃OD): δ 8.66, 8.16-8.20, 7.99, 7.63-7.67, 7.57, 7.29, 6.98, 5.74, 5.32-5.40, 4.32-4.39, 4.19-4.27, 3.52-3.65, 2.88-3.01, 2.72, 2.11-2.35, 1.64-1.72.

### Example 10-3: 2-({2-[(2R)-2-{(1E,3S)-4-[3-(Benzoylamino)phenyl]-3-hydroxy-1-buten-1-yl}-5-oxo-1-pyrrolidinyl]ethyl}thio)-1,3-thiazole-4-carboxylic acid

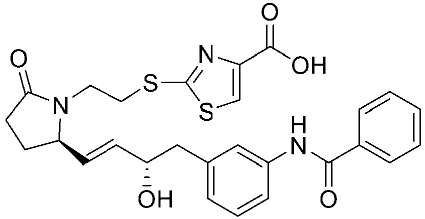

HPLC retention time (min): 0.943;
¹H-NMR (CDCl₃): δ 8.32, 8.08, 8.02, 7.87-7.94, 7.82, 7.44-7.57, 7.27-7.37, 6.94-7.00, 5.76-5.87, 5.51, 4.43, 4.08-4.17, 3.57-3.68, 3.14-3.42, 3.06-3.12, 2.75-2.90, 2.28-2.44, 2.15-2.25, 1.68-1.77, 1.49-1.67, 1.26-1.37.

### Example 10-4: 2-[(2-{(2R)-2-[(1E,3S)-4-{3-[(Ethylcarbamoyl)amino]phenyl}-3-hydroxy-1-buten-1-yl]-5-oxo-1-pyrrolidinyl}ethyl)thio]-1,3-thiazole-4-carboxylic acid

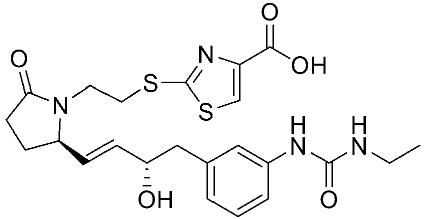

HPLC retention time (min): 0.943;
¹H-NMR (CDCl₃): δ 8.32, 8.08, 8.02, 7.87-7.94, 7.82, 7.44-7.57, 7.27-7.37, 6.94-7.00, 5.76-5.87, 5.51, 4.43, 4.08-4.17, 3.57-3.68, 3.14-3.42, 3.06-3.12, 2.75-2.90, 2.28-2.44, 2.15-2.25, 1.68-1.77, 1.49-1.67, 1.26-1.37.

### Example 11: 2-[({2-[(2-{(2R)-2-[(1E,3S)-3-Hydroxy-8-(2-pyridinylthio)-1-octen-1-yl]-5-oxo-1-pyrrolidinyl}ethyl)thio]-1,3-thiazol-4-yl}carbonyl)amino]ethanesulfonic acid

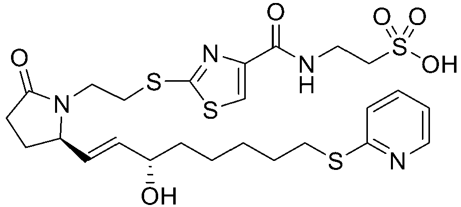

To a DMF (0.3 mL) solution of the compound (27 mg) produced in Example 1, triethylamine (0.02 mL), taurine (CAS No.: 107-35-7, 10 mg), and HATU (22 mg) were added, and the reaction mixture was stirred at room temperature for 2 hours. The reaction mixture was purified by reverse phase column chromatography to give a title compound (14 mg) having the following physical property values.
HPLC retention time (min): 0.786;
¹H-NMR (D₂O): δ 8.27-8.47, 8.01, 7.54-7.72, 7.33-7.51, 5.50, 5.43, 4.22-4.34, 3.88-4.02, 3.67-3.73, 3.53-3.66, 3.31, 3.04-3.16, 2.02-2.41, 1.15-1.77, 1.07.

### Example 12: 2-({[2-({2-[(2R)-2-{(1E,3S)-3-Hydroxy-8-[(3-methoxy-2-pyrazinyl)thio]-1-octen-1-yl}-5-oxo-1-pyrrolidinyl]ethyl}thio)-1,3-thiazol-4-yllcarbonyllamino)ethanesulfonic acid

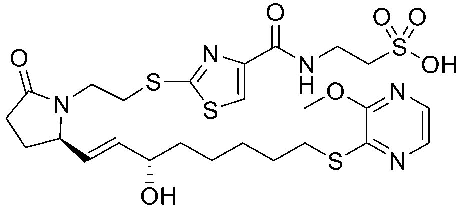

To a DMF (5.4 mL) solution of the compound (540 mg) produced in Example 3, triethylamine (0.56 mL), taurine (188 mg), and HATU (419 mg) were added, and the reaction mixture was stirred at room temperature for 1 hour. The reaction mixture was purified by reverse phase column chromatography to give a title compound (210 mg) having the following physical property values.
HPLC retention time (min): 0.844;
¹H-NMR (D₂O): δ 7.96, 7.85, 7.67, 5.55, 5.41, 4.23-4.29, 3.95-4.01, 3.88, 3.70, 3.59, 3.26, 3.07-3.15, 2.96, 2.24-2.37, 2.10-2.20, 1.63-1.73, 1.40-1.56, 1.23-1.37, 1.05.

### Examples 12-1 to 12-6:

A procedure for a purpose similar to that for Example 11 was carried out by using the corresponding example compound described above in place of the compound produced in Example 1 to give the following example compounds.

### Example 12-1: 2-[({2-[(2-{(2R)-2-[(1E,3S)-3-Hydroxy-8-(2-pyrazinylthio)-1-octen-1-yl]-5-oxo-1-pyrrolidinyl}ethyl)thio]-1,3-thiazol-4-yl}carbonyl)amino]ethanesulfonic acid

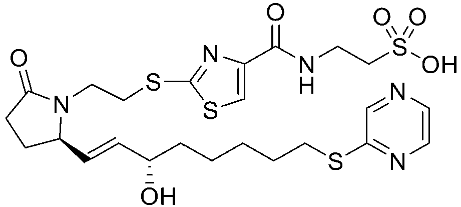

HPLC retention time (min): 0.855;
¹H-NMR (D₂O): δ 8.27-8.47, 8.01, 7.54-7.72, 7.33-7.51, 5.50, 5.43, 4.22-4.34, 3.88-4.02, 3.67-3.73, 3.53-3.66, 3.31, 3.04-3.16, 2.02-2.41, 1.15-1.77, 1.07.

### Example 12-2: 2-({[2-({2-[(2R)-2-{(1E,3S)-3-Hydroxy-7-[(5-methyl-2-pyridinyl)thio]-1-hepten-1-yl}-5-oxo-1-pyrrolidinyl]ethyl}thio)-1,3-thiazol-4-yl]carbonyl}amino)ethanesulfonic acid

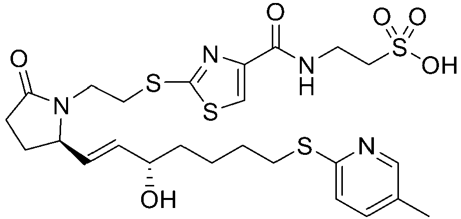

HPLC retention time (min): 0.801;
¹H-NMR (D₂O): δ 8.20, 8.00, 7.84, 7.46, 5.49-5.57, 5.40, 4.27, 3.97, 3.62-3.74, 3.34, 3.13-3.24, 2.99-3.11, 2.21-2.38, 2.04-2.17, 1.50-1.71, 1.44, 1.11-1.32.

### Example 12-3: 2-({[2-({2-[(2R)-2-{(1E,3S)-3-Hydroxy-8-[methyl(phenyl)amino]-1-octen-1-yl}-5-oxo-1-pyrrolidinyl]ethyl}thio)-1,3-thiazol-4-yl]carbonyl}amino)ethanesulfonic acid

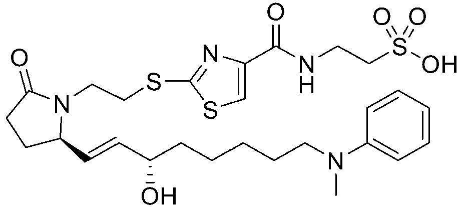

HPLC retention time (min): 0.745;
¹H-NMR (D₂O): δ 7.99, 7.51, 7.44, 5.44, 5.35, 4.20-4.30, 3.81-3.92, 3.66-3.72, 3.56-3.65, 3.44, 3.24-3.33, 2.95-3.16, 2.25, 2.03-2.16, 1.59-1.71, 1.30, 1.04-1.16, 0.82-1.00.

### Example 12-4: 2-({[2-({2-[(2R)-2-{(1E,3S)-3-Hydroxy-8-[(6-methyl-2-pyridinyl)thio]-1-octen-1-yl}-5-oxo-1-pyrrolidinyl]ethyl}thio)-1,3-thiazol-4-yl]carbonyl}amino)ethanesulfonic acid

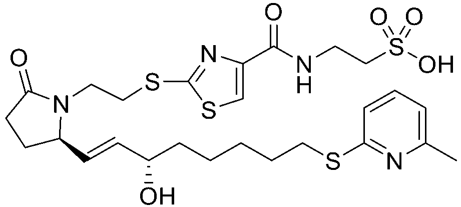

HPLC retention time (min): 0.755;
¹H-NMR (D₂O): δ 7.95-8.03, 7.46, 7.28, 5.52, 5.41, 4.27, 3.97, 3.70, 3.56-3.65, 3.31, 3.05-3.17, 2.53, 2.22-2.39, 2.12, 1.69, 1.55, 1.36-1.46, 1.16-1.36, 1.01-1.11.

### Example 12-5: 2-{[4-({2-[(2R)-2-{(1E,3S)-4-[3-(Benzoylamino)phenyl]-3-hydroxy-1-buten-1-yl}-5-oxo-1-pyrrolidinyl]ethyl}thio)butanoyl]amino}ethanesulfonic acid

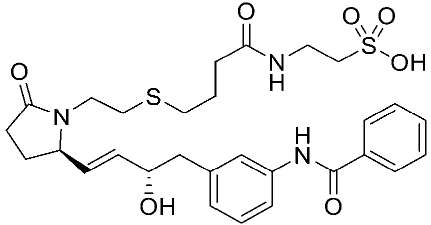

HPLC retention time (min): 0.81;
¹H-NMR (D₂O): δ 7.81, 7.54-7.62, 7.47-7.53, 7.31-7.39, 7.07, 5.65, 5.31, 4.39, 4.09, 3.42, 3.22-3.29, 2.99-3.07, 2.94, 2.73, 2.51-2.58, 2.25-2.36, 2.03-2.20, 1.44-1.67.

### Example 12-6: 2-({[2-({2-[(2R)-2-{(1E,3S)-4-[3-(Benzoylamino)phenyl]-3-hydroxy-1-buten-1-yl}-5-oxo-1-pyrrolidinyl]ethyl}thio)-1,3-thiazol-4-yl]carbonyl}}amino)ethanesulfonic acid

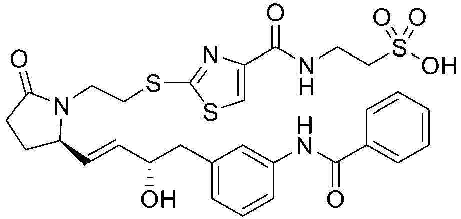

HPLC retention time (min): 0.836;
¹H-NMR (D₂O): δ 7.89, 7.71, 7.49-7.52, 7.41, 7.28-7.34, 6.98-7.05, 5.55-5.64, 5.23-5.33, 4.31-4.43, 4.06-4.18, 3.63, 3.39-3.50, 3.11, 3.03, 2.59-2.75, 2.13-2.35, 1.97-2.10, 1.53-1.69.

### Example 13: (2S)-2-({[2-({2-[(2R)-2-{(1E,3S)-3-Hydroxy-8-[methyl(phenyl)amino]-1-octen-1-yl}-5-oxo-1-pyrrolidinyl]ethyl}thio)-1,3-thiazol-4-yl]carbonyl}amino)pentanedioic acid

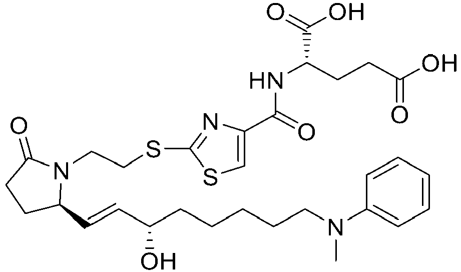

A procedure for a purpose similar to that for Example 11 was carried out by using the compound produced in Example 2 in place of the compound produced in Example 1 and using L-glutamic acid in place of taurine to give a title compound having the following physical property values.
HPLC retention time (min): 0.808;
¹H-NMR (CD₃OD): δ 8.03-8.11, 7.17, 6.76, 6.66, 5.66-5.74, 5.49, 4.60, 4.24-4.31, 4.01, 3.86, 3.42-3.51, 2.86-2.92, 2.28-2.48, 2.14-2.26, 1.75, 1.34-1.62, 1.22-1.34.

### Example 14: (2R)-2-({[2-({2-[(2R)-2-{(1E,3S)-3-Hydroxy-8-[methyl(phenyl)amino]-1-octen-1-yl}-5-oxo-1-pyrrolidinyl]ethyl}thio)-1,3-thiazol-4-yl]carbonyl}amino)pentanedioic acid

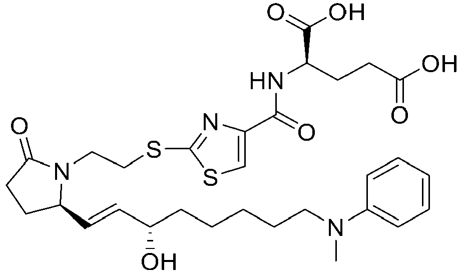

A procedure for a purpose similar to that for Example 11 was carried out by using the compound produced in Example 2 in place of the compound produced in Example 1 and using D-glutamic acid in place of taurine to give a title compound having the following physical property values.
HPLC retention time (min): 0.802;
¹H-NMR (CD₃OD): δ 8.10, 8.03-8.07, 7.17, 6.73-6.78, 6.66, 5.68-5.75, 5.48, 4.60, 4.25-4.32, 4.00, 3.85, 3.40-3.53, 2.89-2.92, 2.27-2.48, 2.14-2.25, 1.75, 1.34-1.60, 1.22-1.33.

### Pharmacological Example 1: Measurement of agonist activity on various human prostanoid receptors

Using CHO cells or Chem1 cells (EP1-CHO, EP2-CHO, EP3-Chem1, EP4-CHO, and IP-CHO) in which various human prostanoid receptors were forcibly expressed, the agonist activity of the test compound on various prostanoid receptors was examined using the intracellular calcium concentration for EP1 and EP3 and the intracellular cyclic AMP (hereinafter, it is abbreviated as cAMP) production amount for EP2, EP4, and IP as indices.

### <Compound treatment>

The test compound and control substances (PGE2 and iloprost) were dissolved in dimethyl sulfoxide (DMSO) to prepare a 10 mmol/L solution. The prepared 10 mmol/L solution was thawed at the time of use, serially diluted using DMSO, diluted with buffer solution for measurement 1 (Hank's balanced salt solution (Thermo Fischer Scientific, Inc.) containing 1 w/v% human serum albumin (Sigma-Aldrich Co. LLC), 2 µmol/L indomethacin (Sigma-Aldrich Co. LLC), 2.5 mmol/L probenecid, and 10 mmol/L HEPES-NaOH (pH 7.4)) or buffer solution for measurement 2 (D-PBS (Nacalai Tesque Inc.) containing 0.1 w/v% human serum albumin, 2 µmol/L diclofenac (Sigma-Aldrich Co. LLC), and 1 mmol/L 3-isobutyl-1-methylxanthine (Sigma-Aldrich Co. LLC)), and subjected to an experiment.

### <Measurement of agonist activity>

### (1) Measurement of EP1 and EP3 activity (measurement of intracellular calcium concentration)

The intracellular calcium concentration was measured using Calcium 6 Assay Kit (Molecular Devices). For EP1-CHO and EP3-Chem1, on the day before measurement, frozen cells were thawed, and the cells were suspended using Ham's F-12 medium (Thermo Fischer Scientific, Inc.) containing inactivated (56°C, 30 min) 9.8 vol% FBS (Thermo Fischer Scientific, Inc.) and penicillin-streptomycin (Nacalai Tesque Inc.), seeded in a 96-well UV plate to 1.0 × 10⁵ cells per well, and static-cultured at 37°C in the presence of 5% CO₂. On the day of measurement, the medium was removed, and 120 µL of a Calcium indicator solution dissolved in buffer solution for measurement 1 was added, and the mixture was incubated at room temperature under light-shielding for about 1 hour. After 1 hour incubation, the 96-well UV plate was set in a fluorescence spectrophotometer (FDSS-7000, Hamamatsu Photonics K.K.) and the intracellular calcium concentration was measured. 30 µL of buffer solution for measurement 1 containing various concentrations of agonists was added, and the mixture was reacted. The intracellular calcium concentration was measured by irradiating the cells with 480 nm excitation light and measuring the fluorescence intensity at 540 nm.

### (2) Measurement of EP2, EP4, and IP agonist activity (measurement of cAMP concentration)

For EP2-CHO, EP4-CHO, and IP-CHO, on the day of measurement, frozen cells were thawed, and the cells were suspended in D-PBS containing 2 µmol/L diclofenac, and centrifuged at 500 g for 3 minutes at room temperature, and then the supernatant was removed. The cells were suspended in buffer solution for measurement 2, and 10 µL each of the suspension was dispensed into a 384-well plate so that the number of cells per well was 2.0 × 10⁵ (EP2), 5.0 × 10⁴ (EP4), and 1.0 × 10⁵ (IP). 20 µL of buffer solution for measurement 2 containing various concentrations of agonists was added, and the mixture was reacted at room temperature for 1 hour. The cAMP concentration was measured using a cAMP HTRF HiRange kit (CIS Bio International S.A.). According to the two step protocol of the kit instruction, 5 µL each of cAMP-D2 and cryptase diluted with lysis buffer was added, and the mixture was incubated at room temperature for 1 hour. After incubation for 1 hour, time-resolved fluorescence at 620 nm and 660 nm when excited at 340 nm was measured using Synergy Neo (Bio teck), and the ratio (TRF ratio) was determined to calculate the cAMP concentration from the calibration curve.

### <Result>

The agonist activity of the compound of the present disclosure on various prostanoid receptors was measured using the above method. The measurement results of the agonist activity (EC₅₀ value) for an EP4 receptor are shown in Table 1, and the measurement results of the agonist activity (EC₅₀ value) for EP1, EP2, EP3, and IP receptors are shown in Table 2. The compounds of the present disclosure described in the tables were confirmed to have potent EP4 receptor agonist activity and selectivity for EP1, EP2, EP3, and IP.

**[Table 1]**

| Example | EC50 (µmol/L) |
|---|---|
| | EP4 |
| 1 | 0.03 |
| 1-1 | 0.04 |
| 2 | 0.08 |
| 3 | 0.04 |
| 5 | 0.32 |
| 10 | 0.03 |

**[Table 2]**

| Example | EC50 (µmol/L) | | | |
|---|---|---|---|---|
| | EP1 | EP2 | EP3 | IP |
| 1 | >10 | 0.92 | >10 | >10 |
| 1-1 | >10 | 0.26 | >10 | >10 |
| 2 | >10 | 1.30 | >10 | >10 |
| 3 | >10 | 1.37 | >10 | >10 |
| 5 | >10 | 6.00 | 9.20 | >10 |
| 10 | >10 | >10 | >10 | >10 |

### Pharmacological Example 2: Calculation of large intestine selectivity by mouse PK

The test compound was dissolved in an aqueous solution of 0.5% methyl-cellulose and administered to C57BL/6 mice at 1, 3, or 10 mg/kg by an oral administration method. Blood samples at various time points after administration (after 0.5, 1, 2, 4, 7, or 9 hours) were centrifuged at 12000 rpm for 5 minutes to collect plasma. A large intestine was collected, washed with physiological saline, and homogenized with purified water in an amount of 4 times the weight of the large intestine. Plasma and large intestine concentrations of the test compound were determined by LC/MS/MS analysis. Large intestine selectivity was determined as a ratio of the highest large intestine concentration to the highest plasma concentration. For example, the compounds produced in Example 1, Example 2, Example 3, and Example 10 showed 302-fold, 440-fold, 391-fold, and 620-fold large intestine selectivity, respectively. In addition, the carboxylic acid forms (compounds produced in Example 1, Example 2, and Example 10, respectively) when the compounds produced in Example 11, Example 12-3, and Example 12-5 were administered exhibited 248-fold, 318-fold, and 395-fold large intestine selectivity, respectively.

On the other hand, when the large intestine selectivity of the compound of Example 6 (22) described in Patent Literature 3 (Comparative Compound A) and the compound of Example 6 (92) described in Patent Literature 3 (Comparative Compound B) having the following structures as comparative compounds was measured, the large intestine selectivity was 20 times and 133 times.

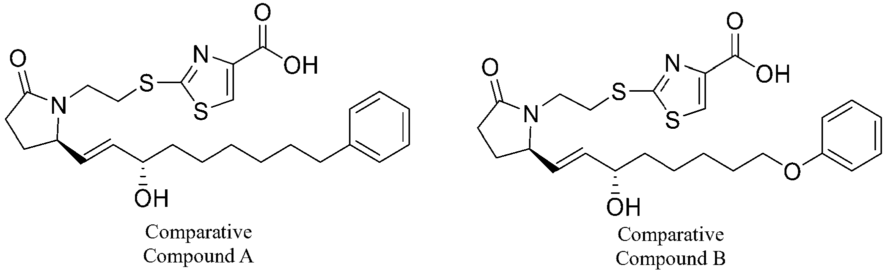

### Pharmacological Example 3: Dextran sodium sulfate (DSS)-induced ulcerative colitis mouse model

Adult C57BL/6 mice were used in this assay. The day on which 2% DSS drinking water started was defined as Day 0. A DSS solution was placed in a transparent plastic water bottle, and the mice were allowed to freely drink from Day 0 to Day 7 via a metal nozzle to induce ulcerative colitis. The test substance was orally administered once a day at a dose of 10 mL/kg for 7 days from Day 0 to Day 6. Stool conditions were observed every day from Day 0 to Day 7, and a disease activity index (DAI) was evaluated according to a reference score (Diarrhea score 0: normal stool, 1: loose stool, 2: diarrhea stool, 3: watery stool. Bloody stool score 0: normal stool, 1: brown mild bloody stool, 2: reddish color in stool, 3: melena); DAI score = diarrhea score + bloody stool score plus the sum of both scores.

The compounds of the present disclosure were tested in this assay. Efficacy in this model is evidenced by a decrease in the DAI score when compared to that of vehicle-treated animals (control group). The compounds of Example 1 (0.3 mpk), Example 2 (0.3 mpk), Example 10 (1 mpk), Example 11 (0.3 mpk), Example 12 (3 mpk), Example 12-3 (0.3 mpk), and Example 12-5 (3 mpk) showed a 50% suppression of the control group score in the DSS colitis mouse model at a dose of 0.3, 1, or 3 mg/kg.

### Pharmacological Example 4: Calculation of concentration acting on circulatory organ by mouse tail cuff and calculation of safety margin

The action on the mouse circulatory organ under awakening can be evaluated by a tail cuff method using a non-preheating, non-invasive blood pressure monitor (MK-2000). A cuff pulse sensor is attached to the tail of a mouse acclimatized in advance to the measurement operation, and the systolic blood pressure calculated from the pulse wave of the tail artery is measured. The measurement is continuously performed a plurality of times, and an average value thereof is adopted. The test substance is orally administered once at a dose of 10 mL/kg. The blood pressure is measured before administration of the test substance and after a certain period of time from administration, and a case where the post-administration systolic blood pressure varies by 10% or more as compared with the pre-administration systolic blood pressure is determined as an influence on the circulatory organ. Blood is collected immediately after blood pressure measurement, and the concentration of the test substance in plasma under the influence on the circulatory organ is defined as the concentration acting on the circulatory organ. The safety margin can be calculated by comparing with the concentration of the test substance at which a significant anti-colitis effect is observed in a mouse model of DSS-induced colitis.

### Pharmacological Example 5: Calculation of safety margin by rat po circulatory organ evaluation test

Regarding an action on the rat circulatory organ under unrestrained conditions and awakening, the test substance was orally administered once to Crl:CD (SD) male rats (4 animals/group) implanted with a telemetry transmitter (HD-S10, Data Sciences International, Inc.) or catheterized in the left carotid artery, and blood pressure (systolic, diastolic, and mean blood pressure) and heart rate were continuously measured. The evaluation time point was set to before administration, 0.5, 1, 2, 3, 4, 7, 12, or 24 hours after administration, and the average value for 10 seconds at the evaluation time point was analyzed.

As a result of testing the compounds of the present disclosure in this assay, the no observed effect level on the rat circulatory organ (blood pressure and heart rate) was 30 mg/kg for all of the compounds of Example 11, Example 12, Example 12-3, and Example 12-5. The safety margin (dose basis) of the compounds of the present disclosure was calculated to be 100 times for the compound of Example 11, 10 times for the compound of Example 12, 100 times for the compound of Example 12-3, and 10 times for the compound of Example 12-5 as compared with the effective amount at which a significant anti-colitis effect was observed in the mouse model of DSS-induced colitis described in Pharmacological Example 3. From the above results, it was considered that the compounds of the present disclosure are compounds having a low risk of toxicity to the circulatory system from the safety margin in which the no observed effect level on the rat circulatory organ was compared with the effective amount of the mouse model.

### Pharmacological Example 6: Calculation of safety margin by monkey po telemetry test

Regarding an action on the monkey circulatory organ under unrestrained conditions and awakening, the test substance was orally administered once to male cynomolgus monkeys (2 animals/dose) implanted with a telemetry transmitter (M11, Data Sciences International, Inc.), and blood pressure (systolic, diastolic, and mean blood pressure) and heart rate were continuously measured. The evaluation time point was set to before administration, 0.5, 1, 2, 4, 8, 10, 12, 16, or 24 hours after administration, and the average value for 1 minute at the evaluation time point was analyzed.

As a result of testing the compounds of the present disclosure in this assay, the no observed effect level on the monkey circulatory organ (blood pressure and heart rate) was 0.3 mg/kg for the compound of Example 11, 30 mg/kg for the compound of Example 12, and 3 mg/kg for the compound of Example 12-3. The safety margin (dose basis) of the compounds of the present disclosure was calculated to be 1 time for the compound of Example 11, 10 times for the compound of Example 12, and 10 times for the compound of Example 12-3 as compared with the effective amount at which a significant anti-colitis effect was observed in the mouse model of DSS-induced colitis described in Pharmacological Example 3.

On the other hand, as a result of evaluating Comparative Compound B by the same method, the no observed effect level on the monkey circulatory organ (blood pressure and heart rate) was less than 0.01 mg/kg, and the safety margin (dose basis) compared with the effective amount at which a significant anti-colitis effect was observed in the mouse model of DSS-induced colitis described in Pharmacological Example 3 was calculated to be less than 0.03 times. From the above results, it was found that the safety margin, determined by comparing the no observed effect level on the monkey circulatory system with the effective dose in the mouse model, was increased for the compounds of the present disclosure compared to Comparative Compound B.

### [Formulation Examples]

### Formulation Examples

The following components are mixed by a conventional method and compressed to obtain about 10,000 tablets containing 10 mg of the active ingredient in one tablet.

| | |
|---|---|
| · 2-({2-[(2R)-2-{(1E,3S)-3-Hydroxy-8-[methyl(phenyl)amino]-1-octen-1-yl}-5-oxo-1-pyrrolidinyl]ethyl}thio)-1,3-thiazole-4-carboxylic acid | 100 g |
| · Carboxymethyl cellulose calcium (disintegrant) | 20 g |
| · Magnesium stearate (lubricant) | 10 g |
| · Microcrystalline cellulose | 870 g |

### INDUSTRIAL APPLICABILITY

The compound of the present disclosure is useful as an EP4 agonist, in particular, it has EP4 agonist activity and selectivity for other subtypes. Furthermore, the compound of the present disclosure is excellent in large intestine selectivity and thus has a low risk of toxicity to the circulatory system, and therefore a compound of the present disclosure as an active ingredient is useful as a preventive and/or therapeutic agent for a disease related to an EP4 receptor.

## Claims

1. A compound represented by general formula (I): 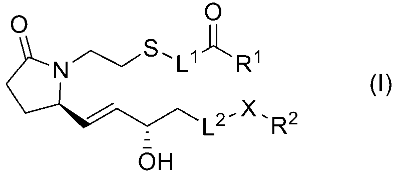 wherein
R¹ represents (1) an -OH group, (2) an -NHCH₂CH₂SO₃H group, (3) an - NHCH₂COOH group, or (4) an -NHCH(COOH)CH₂CH₂COOH group;
L¹ represents (1) a thiazole ring or (2) C1-4 alkylene;
L² represents (1) C3-5 alkylene or (2) a benzene ring;
X represents (1) -CO-, (2) -NR^{X}-, or (3) a sulfur atom;
R^{X} represents (1) a hydrogen atom or (2) a C1-4 alkyl group;
R² represents (1) a methyl group, (2) a benzene ring, (3) a 5- to 10-membered heterocyclic ring, (4) a -COR²⁻¹ group, (5) a -CO-(benzene ring) group, (6) a -CO-(5- to 10-membered heterocyclic ring) group, (7) an -NH-(benzene ring) group, or (8) an -OH group;
when R² represents a benzene ring, a 5- to 10-membered heterocyclic ring, a -CO-(benzene ring) group, a -CO-(5- to 10-membered heterocyclic ring) group, or an -NH-(benzene ring) group, the benzene ring or the 5- to 10-membered heterocyclic ring may be substituted with 1 to 5 R²⁻²s;
R²⁻¹ represents (1) a C1-4 alkyl group that may be substituted with a C1-4 alkoxy group or (2) an -NH-(C1-4 alkyl) group; and
R²⁻² represents (1) a halogen atom, (2) a C1-4 alkyl group, (3) a C1-4 haloalkyl group, (4) a C1-4 alkoxy group, (5) a C1-4 haloalkoxy group, or (6) a benzene ring; or a pharmaceutically acceptable salt thereof.

2. The compound according to claim 1 or a pharmaceutically acceptable salt thereof, wherein L¹ is a thiazole ring, and the thiazole ring is represented by: 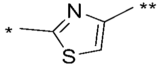 wherein * represents a bonding site with a sulfur atom, and ** represents a bonding site with -COR¹.

3. The compound according to claim 1 or a pharmaceutically acceptable salt thereof, wherein the 5- to 10-membered heterocyclic ring in R² is a heterocyclic ring selected from the group consisting of (1) a pyridine ring, (2) a pyrazine ring, (3) a pyrimidine ring, (4) an imidazole ring, (5) a thiazole ring, (6) a triazole ring, (7) a tetrazole ring, (8) a furan ring, (9) a quinoline ring and (10) a pyrazole ring.

4. The compound according to claim 1 or a pharmaceutically acceptable salt thereof, which is represented by general formula (I-1): 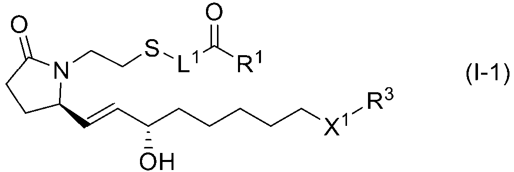
wherein R³ represents (1) a benzene ring, (2) a pyridine ring, (3) a pyrazine ring, (4) a pyrimidine ring, (5) an imidazole ring, (6) a thiazole ring, (7) a triazole ring, (8) a tetrazole ring, (9) a furan ring, (10) a quinoline ring, or (11) a pyrazole ring;
R³ may be substituted with 1 to 5 R³⁻¹s;
R³⁻¹ represents (1) a halogen atom, (2) a C1-4 alkyl group, (3) a C1-4 haloalkyl group, (4) a C1-4 alkoxy group, (5) a C1-4 haloalkoxy group, or (6) a benzene ring;
X¹ represents (1) -NR^{X}- or (2) a sulfur atom; and
the other symbols represent the same meaning as the symbols described in claim 1.

5. The compound according to claim 4 or a pharmaceutically acceptable salt thereof, which is represented by general formula (I-2): 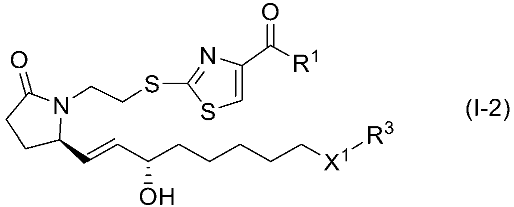 wherein all symbols represent the same meaning as the symbols described in claim 4.

6. The compound according to claim 1 or a pharmaceutically acceptable salt thereof, which is represented by general formula (I-4): 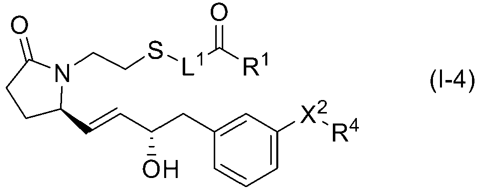
wherein R⁴ represents (1) a -COR⁴⁻¹ group, (2) a -CO-(benzene ring) group, (3) a - CO-(pyridine ring) group, (4) a -CO-(pyrazine ring) group, (5) a -CO-(furan ring) group, (6) an -NH-(benzene ring) group, or (7) an -OH group;
when R⁴ represents a -CO-(benzene ring) group, a -CO-(pyridine ring) group, a -CO-(pyrazine ring) group, a -CO-(furan ring) group, or an -NH-(benzene ring) group, the benzene ring, the pyridine ring, the pyrazine ring, or the furan ring may be substituted with 1 to 5 R⁴⁻²s;
R⁴⁻¹ represents (1) a C1-4 alkyl group that may be substituted with a C1-4 alkoxy group or (2) an -NH-(C1-4 alkyl) group;
R⁴⁻² represents (1) a halogen atom, (2) a C1-4 alkyl group, (3) a C1-4 haloalkyl group, (4) a C1-4 alkoxy group, (5) a C1-4 haloalkoxy group, or (6) a benzene ring;
X² represents (1) -CO- or (2) -NR^{X}-; and
the other symbols represent the same meaning as the symbols described in claim 1.

7. The compound according to claim 1, wherein the compound represented by general formula (I) is a compound selected from the group consisting of:
(1) 2-[(2-{(2R)-2-[(1E,3S)-3-hydroxy-8-(2-pyridinylthio)-1-octen-1-yl]-5-oxo-1-pyrrolidinyl}ethyl)thio]-1,3-thiazole-4-carboxylic acid;
(2) 2-[(2-{(2R)-2-[(1E,3S)-3-hydroxy-8-(2-pyrazinylthio)-1-octen-1-yl]-5-oxo-1-pyrrolidinyl}ethyl)thio]-1,3-thiazole-4-carboxylic acid;
(3) 2-({2-[(2R)-2-{(1E,3S)-3-hydroxy-8-[methyl(phenyl)amino]-1-octen-1-yl}-5-oxo-1-pyrrolidinyl]ethyl}thio)-1,3-thiazole-4-carboxylic acid;
(4) 2-({2-[(2R)-2-{(1E,3S)-3-hydroxy-8-[(3-methoxy-2-pyrazinyl)thio]-1-octen-1-yl}-5-oxo-1-pyrrolidinyl]ethyl}thio)-1,3-thiazole-4-carboxylic acid;
(5) 2-[(2-{(2R)-2-[(1E,3S)-3-hydroxy-9-(2-pyridinylthio)-1-nonen-1-yl]-5-oxo-1-pyrrolidinyl}ethyl)thio]-1,3-thiazole-4-carboxylic acid;
(6) 4-({2-[(2R)-2-{(1E,3S)-4-[3-(benzoylamino)phenyl]-3-hydroxy-1-buten-1-yl}-5-oxo-1-pyrrolidinyl]ethyl}thio)butanoic acid;
(7) 2-[({2-[(2-{(2R)-2-[(1E,3S)-3-hydroxy-8-(2-pyridinylthio)-1-octen-1-yl]-5-oxo-1-pyrrolidinyl}ethyl)thio]-1,3-thiazol-4-yl}carbonyl)amino]ethanesulfonic acid;
(8) 2-({[2-({2-[(2R)-2-{(1E,3S)-3-hydroxy-8-[(3-methoxy-2-pyrazinyl)thio]-1-octen-1-yl}-5-oxo-1-pyrrolidinyl]ethyl}thio)-1,3-thiazol-4-yl]carbonyl}amino)ethanesulfonic acid;
and
(9) 2-{[4-({2-[(2R)-2-{(1E,3S)-4-[3-(benzoylamino)phenyl]-3-hydroxy-1-buten-1-yl}-5-oxo-1-pyrrolidinyl]ethyl}thio)butanoyl]amino}ethanesulfonic acid; or a pharmaceutically acceptable salt thereof.

8. A pharmaceutical composition comprising the compound according to any one of claims 1 to 7, or a pharmaceutically acceptable salt thereof, as an active ingredient, and further comprising a pharmaceutically acceptable carrier.

9. The pharmaceutical composition according to claim 8, which is an EP4 agonist.

10. The pharmaceutical composition according to claim 8, which is a therapeutic and/or preventive agent for a disease related to an EP4 receptor.

11. The pharmaceutical composition according to claim 10, wherein the disease related to an EP4 receptor is immune disease, asthma, neuronal cell death, arthritis, lung injury, pulmonary fibrosis, pulmonary emphysema, bronchitis, chronic obstructive pulmonary disease, liver injury, acute hepatitis, nephritis, renal failure, hypertension, myocardial ischemia, systemic inflammatory response syndrome, sepsis, hemophagocytic syndrome, macrophage activation syndrome, Still's disease, Kawasaki disease, burn injury, systemic granuloma, inflammatory bowel disease, hypercytokinemia during dialysis, multiple organ failure, and/or shock.

12. The pharmaceutical composition according to claim 10, wherein the disease related to an EP4 receptor is inflammatory bowel disease, and the inflammatory bowel disease is ulcerative colitis or Crohn's disease.

13. A therapeutic and/or preventive agent for a disease related to an EP4 receptor, the therapeutic and/or preventive agent comprising the compound according to any one of claims 1 to 7 or a pharmaceutically acceptable salt thereof.

14. A method for preventing and/or treating a disease related to an EP4 receptor, the method comprising administering the compound according to any one of claims 1 to 7 or a pharmaceutically acceptable salt thereof, or a pharmaceutical composition containing the compound according to any one of claims 1 to 7 or a pharmaceutically acceptable salt thereof as an active ingredient and further a pharmaceutically acceptable carrier to a patient in need of prevention and/or treatment of a disease related to an EP4 receptor.

15. A compound according to any one of claims 1 to 7 or a pharmaceutically acceptable salt thereof, for use in prevention and/or treatment of a disease related to an EP4 receptor.

16. Use of the compound according to any one of claims 1 to 7 or a pharmaceutically acceptable salt thereof, for the manufacture of a preventive and/or therapeutic agent for a disease related to an EP4 receptor.
